# EUROPÄISCHE PATENTSCHRIFT

(11) **EP 2 208 217 B1**
(45) Veröffentlichungstag und Bekanntmachung des Hinweises auf die Patenterteilung: **16.09.2015**
(21) Anmeldenummer: 08786486.4
(22) Anmeldetag: 26.07.2008
(51) Int. Cl.: B05D 7/14, H01J 37/32, B05D 3/14, B29C 59/10, B29C 59/12, B29C 59/14

(54) **VERFAHREN UND VORRICHTUNG ZUR PLASMAGESTÜTZTEN OBERFLÄCHENBEHANDLUNG**
METHOD AND DEVICE FOR THE PLASMA-AIDED TREATMENT OF SURFACES
PROCEDE ET DISPOSITIF POUR LE TRAITEMENT DE SURFACE A BASE DE PLASMA

(30) Priorität: 08.08.2007 DE 102007037406
(43) Veröffentlichungstag der Anmeldung: 21.07.2010
(73) Patentinhaber: Neoplas GmbH, 17489 Greifswald (DE)
(72) Erfinder: EHLBECK, Joerg, 17498 Hinrichshagen (DE); FOEST, Ruediger, 17498 Neuenkirchen (DE); KINDEL, Eckhard, 17489 Greifswald (DE); LEMBKE, Norbert, 17489 Greifswald (DE); STIEBER, Manfred, 17489 Greifswald (DE); WELTMANN, Klaus-Dieter, 18609 Binz (DE)
(74) Vertreter: Wehlan, Helmut
(86) Internationale Anmeldenummer: PCT/EP2008/059840
(87) Internationale Veröffentlichungsnummer: WO 2009/019156

(56) Entgegenhaltungen:
- EP-A- 0 355 622
- WO-A-93/05890
- DE-A1-102005 001 264
- DE-U1-202004 016 083
- US-A1- 2006 040 067
- US-A1- 2007 159 517

## Beschreibung

Die Erfindung betrifft ein Verfahren und eine Reihe von Vorrichtungen zur trockenen Reinigung, Aktivierung, Beschichtung, Modifizierung und biologischen Dekontamination (Entkeimung, Desinfektion, Sterilisation) von Oberflächen mittels eines, durch eine so genannte Oberflächen-Barrierenentladung erzeugten, Atmosphärendruckplasmas.

### Stand der Technik

Zur Behandlung von Materialoberflächen mit dem Ziel, die Oberfläche zu reinigen (bzw. zu dekontaminieren), aktivieren, funktionalisieren bzw. beschichten, um sie für nachfolgende technologische Prozesse, wie beispielsweise Kleben, Drucken, Lackieren vorzubereiten bzw. um Arbeiten unter keimfreien Bedingungen durchführen zu können, werden bereits standardmäßig plasmatechnische Verfahren eingesetzt. In der Vergangenheit kamen dafür, insbesondere für komplexe Oberflächengeometrien, vorrangig Niederdruckplasmen in Betracht. Wegen der hohen Anlagenkosten für die dafür erforderlichen Vakuumapparaturen, der diskontinuierlichen Arbeitsweise sowie wegen der durch die Größe des verwendeten Rezipienten eingeschränkten geometrischen Abmessungen der zu behandelnden Werkstücke ist ein Einsatz von Niederdruck-Plasmaverfahren für großtechnische Anwendungen, insbesondere in der industriellen Fließfertigung nur bedingt möglich. Für die Integration plasmatechnologischer Verfahren der Oberflächenbehandlung in industriellen Fertigungsstrecken werden Atmosphärendruckplasmen genutzt. Plasmen dieser Art können beispielsweise durch Korona- oder Barrierenentladungen erzeugt werden. Sie können aber auch in Form von Normaldruck-Strahlplasmen verwendet werden, die auf der Grundlage von Korona-, Barrieren- oder Bogenentladungen durch die Realisierung geeigneter Prozessgasströmungen generiert werden.

Verfahren und Vorrichtungen zur Oberflächenbehandlung, die auf der Nutzung derartiger Plasmen basieren, werden in zahlreichen Druckschriften beschrieben und teilweise auch bereits für unterschiedliche Anwendungen genutzt. Die in diesen Schriften beschriebenen technischen Lösungen sind aber mindestens mit einem oder mit mehreren der folgenden Nachteile verbunden:
- Es werden aufwendige, kostspielige Stromversorgungsgeräte benötigt.
- Durch einen hohen Energieverbrauch sowie teilweise auch durch hohen Gasverbrauch und erforderliche Kühlung entstehen relativ hohe Betriebskosten.
- Eine gleichmäßige Behandlung (insbesondere Beschichtung) des Materials ist durch die inhomogene Struktur der Plasmen erschwert.
- In vielen Fällen sind die Entladungen inhomogen und bestehen aus vielen kleinen, energiereichen Mikroentladungen, die zu lokalen Materialschäden führen können.
- Die Vorrichtungen sind nicht universell an beliebigen Werkstücken anwendbar. Entweder sind sie nur für die Behandlung von ebenen Materialien mit einer auf einige Millimeter begrenzten Materialstärke (wie beispielsweise Folien und Bahnmaterialien) geeignet oder nur mittels aufwändiger Positionierssysteme an Werkstücke mit komplexer Oberflächengeometrie adaptierbar.

Die Vorrichtungen sind nicht als Handgeräte für den manuell geführten Betrieb nutzbar.

Zum Stand der Technik gehören auch die nachfolgend aufgeführten Druckschriften. In DE 195 32 412 A1 wird ein Verfahren zur Oberflächen-Vorbehandlung von Werkstücken mittels eine Plasmastrahls beschrieben, wobei der Plasmastrahl zunächst als Lichtbogen in einer Düse mit Elektroden erzeugt und durch eine verwirbelte Arbeitsgasströmung aus der Elektrodenanordnung heraus auf das zu behandelnde Werkstück übertragen wird. In DE 298 05 999 U1 wird eine Vorrichtung beschrieben, die mittels eines Rotationskopfes mit mindestens einer wie oben beschriebenen, exzentrisch angeordneten Plasmadüse einen parallel zur Rotationsachse gerichteten Plasmastrahl trägt, Oberflächen plasmabehandeln kann. In DE 10 2004 033 728 A1 wird ein Verfahren zum Bearbeiten und Verkleben von Werkstücken aus einem Metall oder einer Metalllegierung mit einer hydratisierten Oxid- und/oder Hydroxidschicht beschrieben, wobei die Reinigung, Aktivierung und Nachbehandlung mit einem atmosphärischen Plasmastrahl erfolgen. DE 199 27 557 A1 beschreibt ein Verfahren zur Vorbehandlung von zu schweißenden oder zu lötenden Werkstücken, wobei zwischen einer Elektrode und der zu behandelnden Oberfläche des Werkstücks eine Hochfrequenz-Bogenentladung erzeugt wird. Die hier beschriebenen Vorrichtung und Verfahren weisen wesentliche Unterschiede zu den später beschriebenen erfindungsgemäßen Verfahren und Vorrichtungen auf. Die Elektrodenanordnung ist stets so ausgeführt, dass sich die Elektroden an oder in unmittelbarer Nähe zum Werkstück befinden und das Plasma möglichst direkt an seinem Wirkungsort erzeugt wird. Die eingesetzte Gasströmung hat nicht die Funktion, das Plasma aus der Elektrodenanordnung heraus an das Werkstück zu tragen oder die Elektrodenanordnung zu kühlen, wie im Falle der Plasmastrahlen oder Plasmajets, sondern dient lediglich der lokalen Intensivierung des Plasmas an seinem Wirkungsort und der Steuerung seiner Parameter (u.a. Art und Zustand der angeregten Spezies). Durch die spezielle Elektrodenanordnung wird der Gasverbrauch sehr gering gehalten und die zum Plasmabetrieb erforderliche Zündspannung minimiert. So können die benötigten Stromversorgungsgeräte sehr klein, einfach und kompakt gestaltet werden.

In den Druckschriften DE 43 25 939 C1, WO 2004/053185 A1 und DE 38 27 629 A1 werden Verfahren und Vorrichtungen zur Oberflächenvorbehandlung beschrieben, die auf dem Prinzip der sog. Volumen-Barrierenentladung (auch stille Entladung, dielektrisch behinderte Entladung oder Korona-Behandlung) basieren. Die Erfahrung zeigt, dass die Anwendbarkeit sog. Korona-Behandlungsanlagen nur für die Behandlung von ebenen Materialien mit einigen Millimetern Materialstärke (beispielsweise Folien und Bahnmaterialien) praktikabel ist. In WO 2004/053185 A1 wird eine mit einem Dielektrikum bedeckte Elektrode verwendet, um ein Plasma in einer Reaktivgaströmung zu erzeugen um Metalloberflächen, die gleichzeitig als Gegenelektrode fungieren, mittels der Plasmabehandlung zu konservieren. DE 38 27 629 A1 stellt ein Verfahren Oberflächenvorbehandlung von elektrisch leitenden Formmaterialien, wie Metallfolienbahnen oder Kunststofffolien, in deren Polymermatrix elektrisch leitfähige Partikel eingelagert sind, vor. Die Entladung wird zwischen von dielektrischem Material ummantelten Entladungselektroden und den Metallkern einer Walze, die als geerdete Gegenelektrode dient erzeugt, wobei über die Walze die zu behandelnde Folienbahn transportiert wird. Zusätzlich kann das Arbeitsgas mittels einer Zerstäubereinrichtung mit einem schwebfähigen Aerosol versehen werden. In beiden Fällen bilden die Gegenelektrode und die von dielektrischem Material ummantelten Entladungselektroden einen Entladungsspalt, so dass ein sog. kaltes Plasma im Volumen erzeugt wird. In zwei der oben genannten Patenschriften (DE 43 25 939 C1, WO 2004/053185 A1) werden auch sog. indirekte Koronabehadlungen beschrieben. Die in DE 43 25 939 C1 vorgestellte Koronadüse dient der indirekte Plasmabehandlung von bahnförmigen oder profilierten Materialien und weist mindestens zwei Hochspannungselektroden auf, zwischen denen ein oszillierend oder umlaufend geführter Luftstrom austritt. In WO 2004/053185 A1 wird eine sog. indirekte Barrierenentladung zur Konservierung von Metalloberflächen beschrieben. In beiden Fällen hat der Gasstrom, ähnlich wie bei einem Plasmastrahl oder Plasmajet die Aufgabe, die Entladung aus dem Raum zwischen den beiden Elektroden an das zu behandelnde Werkstück heraus zutreiben. Der Gasstrom wirkt somit auf die Form und flächige Gestalt des Plasmas ein. Es werden drei Ausführungen für die rotierende bzw. umlaufende Führung des Luftstroms beschrieben.

In der Patenschrift DE 102 19 197 C1 werden Verfahren und Vorrichtung zur Behandlung der Oberflächen eines Metalldrahts, insbesondere als Beschichtungsvorbehandlung beschrieben. Dabei wird der Metalldrahts an eine Elektrode, die zu dem Metalldraht hin mit einer dielektrischen Abschirmung versehen ist, eine Wechselhochspannung angelegt, um im Gasraum über der Oberfläche des Metalldrahts eine Volumen-Barrierenentladung hervorzurufen.
In der Offenlegungsschrift DE 10 2005 001 264 A1 wird eine Ingredienzanalysevorrichtung und ein Ingredienzanalyseverfahren beschrieben, bei dem Hochfrequenzenergie von einer Energiequelle zugeführt wird, während Heliumgas zu einer, Atmosphärendruck-Plasmaquelle geführt wird, die in der Nähe einer zu analysierenden Substanz angeordnet ist, wobei Plasma erzeugt wird, und die zu analysierende Substanz dem Plasma ausgesetzt ist und Licht emittiert wird. Dabei findet die Plasmaerzeugung nicht auf der Oberfläche, sondern über der Oberfläche statt. Es handelt sich um eine Volumen-Entladung, nicht um eine Oberflächen-Entladung.

### Aufgabe der Erfindung:

Aufgabe der Erfindung ist es, eine Vorrichtung und ein Verfahren zur plasmagestützten Oberflächenbehandlung von Werkstoffen bereitzustellen, die diese Mängel nicht aufweisen.

### Darstellung der Erfindung:

Die Aufgabe wird gemäß den Merkmalen der Patentansprüche 1 und 9 gelöst. Erfindungsgemäß wird eine Vorrichtung und ein Verfahren zur Behandlung von Oberflächen erstmals bereitgestellt, die dadurch gekennzeichnet sind, dass auf der Oberfläche des zu behandelnden Materials bzw. in deren unmittelbarer Nähe eine Oberflächen-Barrierenentladung durch den Kontakt einer dielektrikumbedeckten Hochspannungselektrode mit einer geerdeten, elektrisch leitenden Kontaktelektrode, die in einer, aus einer speziell angepassten Gasdüse strömenden, lokal auf die Kontaktstelle gerichteten, schwachen Prozessgasatmosphäre betrieben wird. Der gerichtete, schwache Prozessgasstrom spielt dabei für die Effizienz der Vorrichtung zur Oberflächenbehandlung, und damit für die Gesamtfunktion, eine wesentliche Rolle. Verwendet man dafür ein Edelgas, wie beispielsweise Argon, so wird die Zündspannung der Oberflächenentladung im Vergleich zu einem Betrieb in Luft deutlich herabgesetzt und die Leuchterscheinung des Plasmas auf der Oberfläche des Isoliermaterials (Dielektrikums) wird nicht nur heller, sondern bedeckt auch eine größere Fläche des Materials. Dieses visuelle Erscheinungsbild ist verbunden mit einem deutlich stärkeren Effekt der Oberflächenbehandlung durch das Plasma, wie beispielsweise durch Kontaktwinkelmessungen und geeignete Methoden der Oberflächendiagnostik nachgewiesen werden kann. Dabei genügt ein sehr geringer Gasfluss, um diesen Effekt zu erzielen. So ist es beispielsweise möglich, mit einer geerdeten Kontaktelektrode von 20 cm Länge und einer daneben angeordneten Breitstrahldüse gleicher Länge, aus der durch einen ca. 0,2 - 0,3 mm schmalen Schlitz das Gas auf die Kontaktstelle strömt, bei einem Gasfluss von 0,5 - 1 slm (slm - Standart Liter pro Minute) Ar bereits einen deutlichen Effekt zu erreichen. Die Vorteile der hier benutzten Oberflächen-Barrierenentladungen gegenüber sogenannten Volumen-Barrierenentladungen, die vor allem darin bestehen, dass sowohl die benötigten Betriebsspannungen, als auch die umgesetzte elektrische Leistung geringer sind und die Plasmen eine homogenere Struktur haben, werden durch die fein dosierte Zuführung dieser Gasströmung noch wesentlich stärker ausgeprägt, so dass sich auch die Anforderungen an die Stromversorgungsgeräte deutlich reduzieren lassen.

Als ein wichtiger Bestandteil der Vorrichtung kann das Stromversorgungsgerät daher mit minimalen geometrischen Abmessungen dimensioniert werden, so dass es beispielsweise in einer als Handgerät ausgeführten Vorrichtung integriert werden kann. Dabei erfolgt die Spannungsversorgung der Hochspannungselektrode durch das schnelle Schalten eines Hochspannungstransformators. Das hierfür erforderliche Schaltsignal mit einer Frequenz von 10 kHz bis 40 kHz und einem variablen Tastverhältnis von 10 bis 90% wird von einem Rechteckgenerator geliefert. Die eigentliche Schaltstufe besteht aus einem Leistungs-MOSFET mit davor geschaltetem Treiberschaltkreis. Die Hochspannungsamplitude ist bis zu einem maximalen Wert von 7 kV_{eff} regelbar.

Überraschenderweise hat sich herausgestellt, das die erfindungsgemäße Vorrichtung ohne Kühlung, mit einem geringen Energie- und Gasverbrauch und mit einem einfachen, kostengünstigen Stromversorgungsgerät betrieben werden kann, eine gleichmäßige, effiziente Behandlung ohne Schädigung des zu behandelnden Materials gestattet, auch für die Behandlung von Werkstücken mit komplexer Oberflächengeometrie geeignet ist und die Möglichkeit bietet, als Handgerät für den manuell geführten Betrieb genutzt zu werden. Die Eigenschaften des Gerätes zeigen Anwendungsgebiete auf, die über den Einsatz zur Oberflächenbehandlung von Werkstücken hinausgehen. So ist beispielsweise bei entsprechender Ausführung eine Behandlung von biologischen Gewebe möglich.

Abhängig von Art, Form und Größe der zu behandelnden Materialien, sowie von dem gewünschten Behandlungseffekt können Aufbau und Anordnung der dielektrikumbedeckten Hochspannungselektrode, der geerdeten Kontaktelektrode und der Gasdüse in unterschiedlicher Weise gestaltet beziehungsweise kombiniert werden. So kann beispielsweise in einem Grundaufbau zur Behandlung von Kunststoffen in Gestalt ebener Folien-, Bahn- bzw. Plattenmaterialien das zu behandelnde Material auf der Isolierstoffschicht einer ebenen, dielektrikumbedeckten Hochspannungselektrode aufgelegt werden. Über die zu behandelnde Kunststoffoberfläche wird die elektrisch leitende, geerdete, mit einer Breitstrahl-Gasdüse gekoppelte Kontaktelektrode geführt. Die Kontaktelektrode kann dabei sowohl als Gleitkontakt, als auch als Rolle oder Pinsel ausgeführt werden und entweder aus Metall oder andere elektrisch leitenden Materialien (zum Beispiel aus elektrisch leitenden Elastomeren) gefertigt sein. Die als schmaler Schlitz ausgebildete Austrittsöffnung der Breitstrahl-Gasdüse ist unmittelbar neben dem Kontaktstreifen der Kontaktelektrode angeordnet. Wird an die Hochspannungselektrode eine Spannung ausreichender Höhe (abhängig von Materialart und -dicke) angelegt, so setzt am Kontaktstreifen auf der Kunststoffoberfläche eine Oberflächenentladung an. Durch den auf den Kontaktstreifen auftreffenden schwachen Gasstrom aus dem Schlitz der Breitstrahldüse wird die Ausdehnung dieser Entladung vergrößert und deren Intensität wesentlich erhöht.

Die Elektrodenanordnung ist stets so ausgeführt, dass sich die Elektroden an oder in unmittelbarer Nähe zum Werkstück befinden und das Plasma möglichst direkt an seinem Wirkungsort erzeugt wird. Die eingesetzte Gasströmung hat nicht die Funktion, das Plasma aus der Elektrodenanordnung heraus an das Werkstück zu tragen oder die Elektrodenanordnung zu kühlen, wie im Falle der Plasmastrahlen oder Plasmajets, sondern dient lediglich der lokalen Intensivierung des Plasmas an seinem Wirkungsort und der Steuerung seiner Parameter (u.a. Art und Zustand der angeregten Spezies). Durch die spezielle Elektrodenanordnung wird der Gasverbrauch sehr gering gehalten und die zum Plasmabetrieb erforderliche Zündspannung minimiert. So können die benötigten Stromversorgungsgeräte sehr klein, einfach und kompakt gestaltet werden.

Außerdem ist eine besonders einfache Handhabung möglich, denn zum einen muss kein bestimmter Abstand zwischen Plasmaquelle und zu behandelnder Oberfläche eingehalten und zum anderen muss dieser nicht mittels aufwändiger Positionierssysteme sichergestellt werden, sondern die Entladung wird in Form eines mobilen Kontaktplasmas einfach über die zu behandelnde Oberfläche geführt. Solche Vorrichtungen sind universell an beliebigen Werkstücken anwendbar. Außerdem gelingt es durch die prinzipiellen Unterschiede im Aufbau stets ein sog. kaltes Plasma zu erzeugen, d.h. die Gastemperatur wird erfahrungsgemäß nur leicht erhöht, im Gegensatz zu den im Stand der Technik beschriebenen Plasmastrahlen, die erfahrungsgemäß bedingt durch die initiale Erzeugung eines Lichtbogens einen nicht unerhebliche Teil der eingekoppelten elektrischen Energie in Wärme umwandeln.

In der hier beschriebenen Erfindung befinden sich - im Unterschied zu den im Stand der Technik genannten Vorrichtungen und Verfahren - die von dielektrischem Material ummantelten Entladungselektroden auf oder am Werkstück, welches die Gegenelektrode darstellt, so dass eine sog. Oberflächen-Barrierenentladung direkt auf der zu behandelnden Oberfläche erzeugt wird. Somit kann die Entladung als mobiles Kontaktplasma einfach über die zu behandelnde Oberfläche geführt werden (Handgerät für manuell geführten Betrieb), wobei die Plasmaausbildung auf der Oberfläche durch eine zusätzliche, aber nur geringe oder seichte Gasströmung (d.h. z.B. keine rotierende bzw. umlaufende Führung des Luftstroms), lediglich noch intensiviert wird. Da die Gasströmung das Plasma nicht mehr an die Oberfläche transportieren muss, ist der Gasverbrauch relativ gering. Diese Unterschiede ermöglichen auch für die Behandlung von sehr komplexen Werkstücken eine sehr einfache Handhabung, die zusätzlich dadurch unterstützt wird, dass aufgrund der Plasmaintensivierung durch die seichte Prozessgasströmung die erforderlichen Stromversorgungsgeräte besonders klein, einfach und kompakt gestaltet werden können.

Im Unterschied zu DE 102 19 197 C1 wird mit der hier beschriebenen Erfindung hingegen ein Verfahren für die äußere Behandlung von isolierten Drähten zur Verbesserung der Benetzbarkeit vorgestellt. Dabei werden die zu behandelnden Drähte als bereits mit einem Dielektrikum ummantelte Hochspannungselektroden genutzt und mittels einer eng an dem zu behandelnden isolierten Draht anliegenden, elektrisch leitenden, geerdeten Kontaktelektrode, angeordnet in einem Rohr aus Isoliermaterial, eine Oberflächen-Barrierenentladung auf der Drahtisolationsoberfläche erzeugt.

Entsprechend dem hier dargestellten Grundaufbau dieser Ausführungsform kann die Vorrichtung als Tischgerät für die Behandlung von ebenen Materialien mit einer eingeschränkten Flächenausdehnung (z.B. in den Formaten DIN A6 bis DIN A0) ausgelegt werden, bei dem die Abtastung der Fläche, ähnlich wie bei einem optischen Scanner, über einen Motorantrieb erfolgt. Geräte dieser Art sind beispielsweise vorteilhaft einsetzbar in Werbeateliers bzw. Druckereien, um durch die Plasmabehandlung von Folien oder Schildern aus Kunststoff eine Verbesserung der Haftung von Klebefolien oder Druckfarben auf diesen Materialien zu erreichen.

Als eine weitere Anwendung bietet diese Grundform der Vorrichtung die Möglichkeit, für große Flächen im Klinikbereich (zum Beispiel in Operationssälen) oder im Lebensmittelbereich, wie beispielsweise für speziell präparierte Tisch- bzw. Wandbeläge mittels dieser Plasmabehandlung eine trockene Reinigung beziehungsweise Desinfektion (Entkeimung, biologische Dekontamination) zu realisieren. Dazu sind die Tisch- bzw. Wandbeläge so zu gestalten, dass sie als dielektrikumbedeckte Hochspannungselektrode genutzt werden können.

Eine weitere Ausführungsform des oben dargestellten Grundaufbaus der Vorrichtung erhält man, wenn anstelle einer ebenen eine als rotierende Walze ausgestaltete, dielektrikumbedeckte Hochspannungselektrode verwendet wird. Mit einer derartigen Vorrichtung lassen sich beispielsweise vorteilhaft Bahnmaterialien oder längere Platten behandeln, die mittels einer geeigneten Vorschubeinrichtung zwischen der rotierenden Hochspannungselektrode und der auf der Oberfläche des Materials gleitenden oder rollenden, als Kontaktelektrode gestalteten Breitstrahl-Gasdüse hindurch bewegt werden.

In einer weiteren Ausführungsform dieser Grundform der Vorrichtung können Hohlkörper aus Kunststoff behandelt werden, indem die Hohlkörper mit einer leitfähigen Masse (beispielsweise mit Stahlwolle, leitfähigem plastischen Material oder mit elektrisch leitender Flüssigkeit) gefüllt werden, die mit der Hochspannung verbunden wird und so gemeinsam mit dem Kunststoff-Hohlkörper als dielektrikumbedeckte Hochspannungselektrode wirkt. Auf der Oberfläche des Hohlkörpers wird durch die manuell darüber geführte Gasdüse mit geerdeter Kontaktelektrode die intensivierte Oberflächenentladung erzeugt.

Eine weitere Ausführungsform dieser Vorrichtung ermöglicht die trockene Reinigung beziehungsweise Entkeimung oder Beschichtung der inneren Oberfläche von Flaschen. Dazu wird die Flasche passgenau von zwei Hälften einer elektrisch leitenden Form umschlossen, die auf Hochspannungspotential gelegt werden. Zusammen mit der Flaschenwand wirkt diese Form als dielektrikumbedeckte Hochspannungselektrode. Im Inneren der Flasche wird eine Flaschenbürste aus elektrisch leitendem Material angeordnet, deren Borsten eng an der inneren Oberfläche der Flasche anliegen und als geerdete Kontaktelektrode wirken. Die Gaszuführung erfolgt dabei über die als Rohr ausgeführte Halterung der Flaschenbürste. Eine Beschichtung der Innenwand der Flasche wird durch die Zumischung eines geeigneten Precursors zum Prozessgas erreicht. Wird als Precursor eine siliziumorganische Verbindung (zum Beispiel Hexamethyldisiloxan HMDSO oder Tetraethylorthosilicat TEOS) verwendet, so können sowohl im Fall ebener Anordnungen als auch im Fall der Innenbehandlung von Flaschen SiOₓ.-Schichten auf den Oberflächen erzeugt werden.

In weiteren Ausführungsformen der Vorrichtung werden eine oder mehrere dielektrikumbedeckte Hochspannungselektroden in einem Handgerät in der Weise integriert, dass die Kontaktflächen zwischen den integrierten, dielektrikumbedeckten Hochspannungselektroden und der geerdeten Kontaktelektrode in der Ebene des Gasaustritts aus der Gasdüse angeordnet sind, so dass in diesem Bereich auf der Oberfläche des Dielektrikums der Hochspannungselektrode eine intensivierte Oberflächenentladung erzeugt wird.

In weiteren Ausführungsformen der Vorrichtung können, wie durch die Zeichnungen zu den Ausführungsbeispielen näher erläutert wird, Katheter und Kabel außen behandelt werden. In ähnlichen Anordnungen können auch nach dem hier dargestellten Arbeitsprinzip Behandlungen und Beschichtungen der inneren Oberfläche von Rohren und Schläuchen realisiert werden.

### Vorteile der Erfindung:

- Das Verfahren ist sehr vielseitig einsetzbar, ist auch für die Behandlung komplexer Oberflächengeometrien geeignet und bietet die Möglichkeit, sowohl in Form eines kostengünstigen Handgerätes, als auch in der Gestalt maschinell gesteuerter Anlagen genutzt zu werden.
- Aufgrund des geringen Energie- und Gasverbrauchs, sowie der geringen Investitionskosten für die Stromversorgungsgeräte und Behandlungseinheiten, bietet das Verfahren eine sehr kostengünstige Lösung für viele Anwendungen.
- Das Verfahren gestattet eine homogene Plasmabehandlung bzw. -beschichtung von Oberflächen, wobei durch eine geeignete Wahl der Prozessparameter Materialschäden durch elektrische Durchschläge oder thermische Belastungen ausgeschlossen werden können.
- Das Verfahren gestattet eine Plasmabehandlung von biologischem Gewebe, wobei durch geeignete Wahl der Prozessparameter elektrische Durchschläge oder thermische Belastungen ausgeschlossen werden können.

Die Erfindung wird nachfolgend anhand von Ausführungsbeispielen näher erläutert, ohne sie auf diese Beispiele zu beschränken.

### Ausführungsbeispiele:

Mit den nachfolgend in verschiedenen Zeichnungen dargestellten Ausführungsbeispielen werden die Erfindung und deren Anwendungsmöglichkeiten detailliert erläutert. Für die Kennzeichnung der einzelnen Elemente des Aufbaus der Vorrichtungen werden folgende Bezugszeichen verwendet:

### Bezugszeichenliste:

| | | | |
|---|---|---|---|
| 1 | Gasdüse | 2 | geerdete Kontaktelektrode |
| 3 | Werkstück | 4 | Isolator (Dielektrikum oder Ferroelektrikum) |
| 5 | Hochspannungselektrode | 6 | Isolation |
| 7 | Hochspannungsquelle | 8 | Gaszufuhr |
| 9 | Oberflächenplasma | 10 | Gehäuse mit Hochspannungsversorgung |
| 11 | Motor mit Magnetkupplung | 12 | Gasaustritt |
| 13 | Bewegungsrichtung | 14 | Fügekante |
| 15 | zweiter Düsenkanal | 16 | Absaugung |
| 17 | Precursor-Zufuhr | 18 | rotierende Bürste |
| 19 | Scharnier | 20 | Griffstück mit Steckeranschluss |

### Kurze Beschreibung der Zeichnungen:

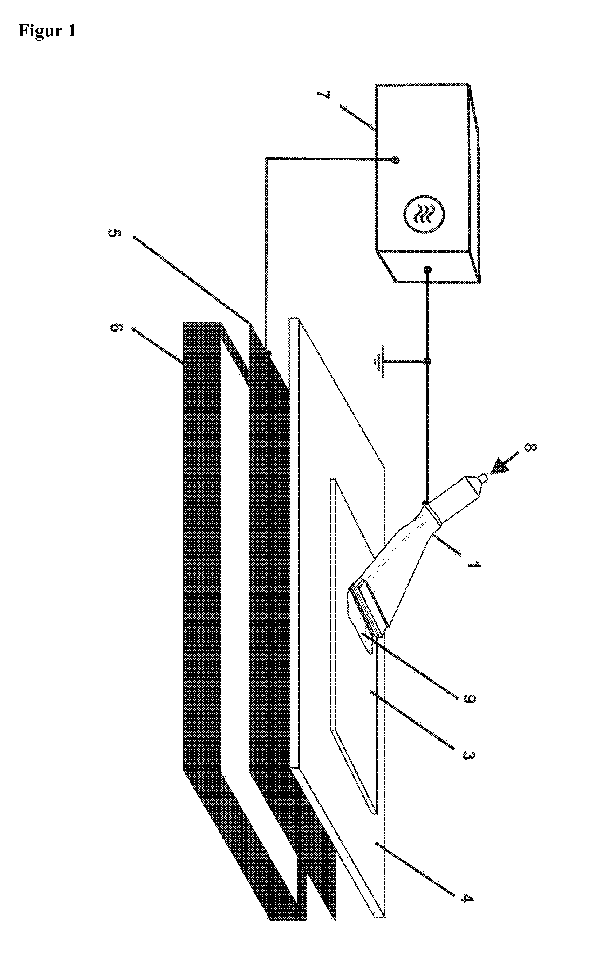

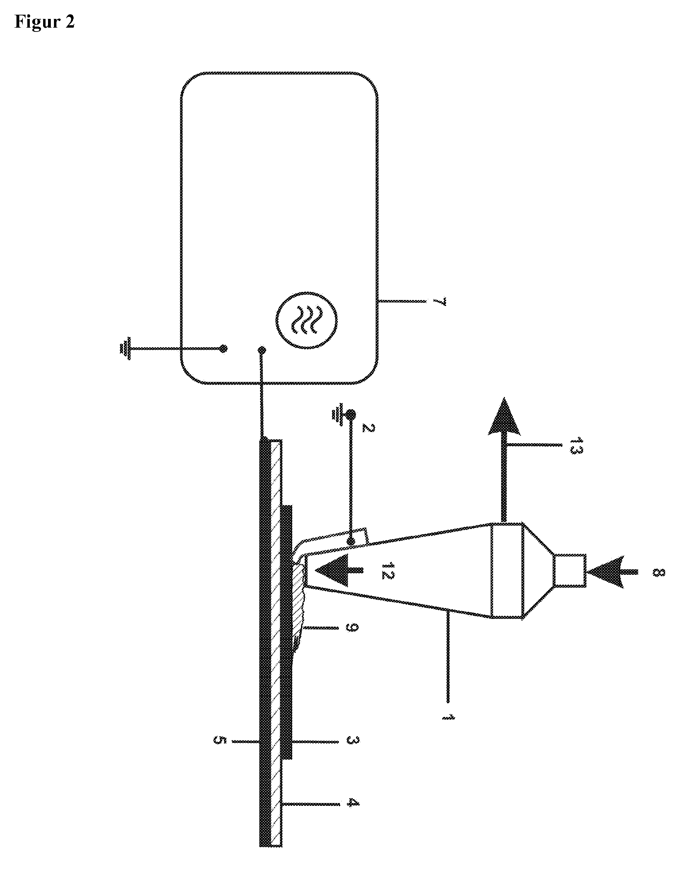

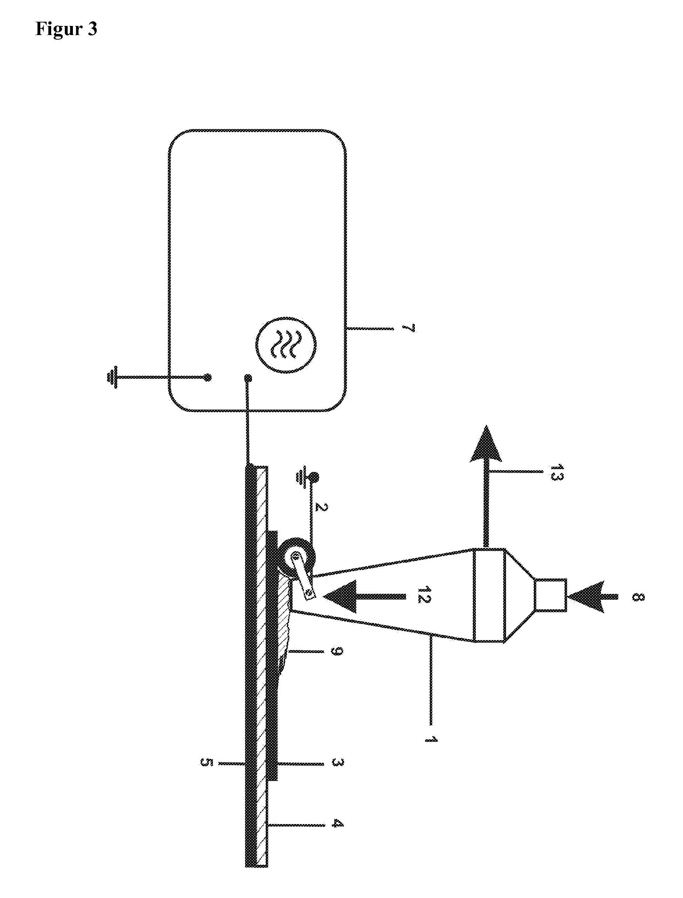

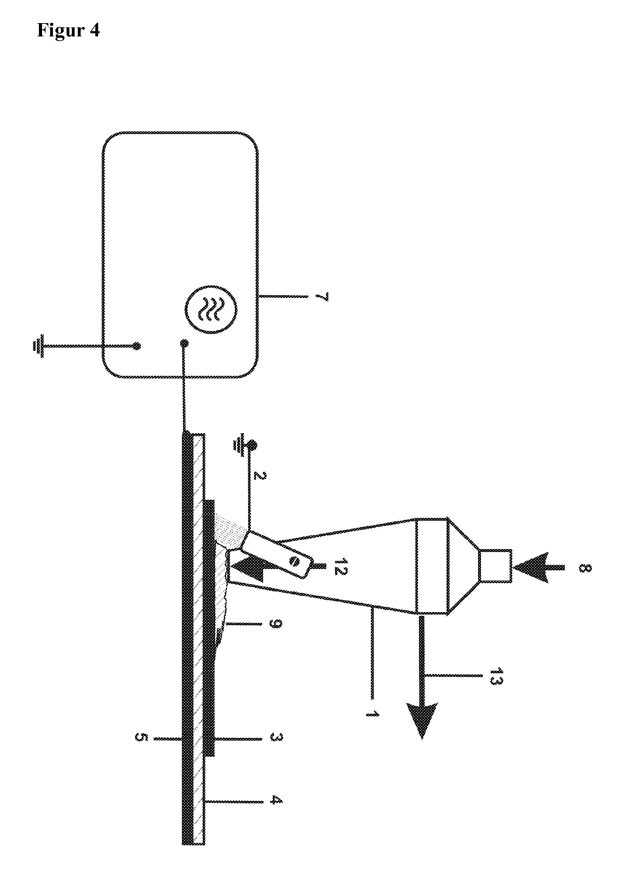

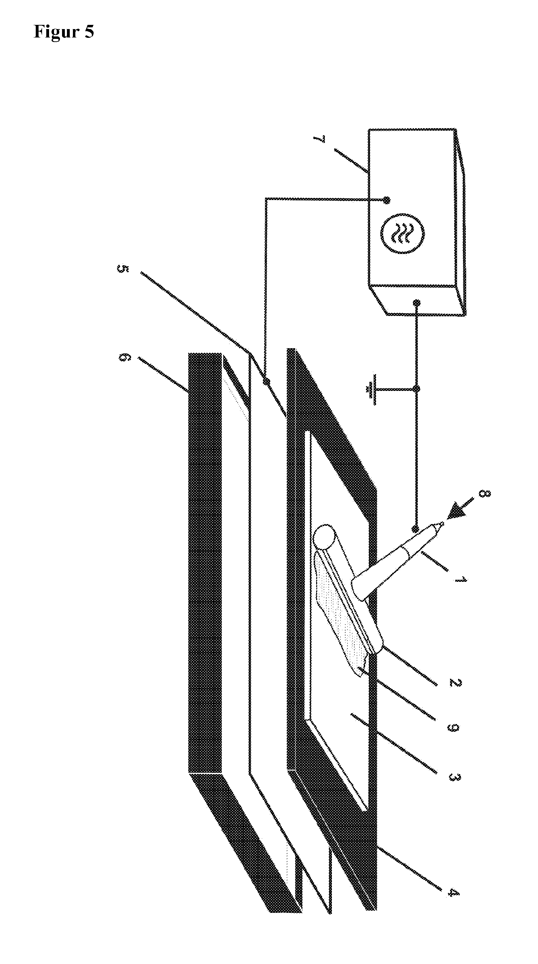

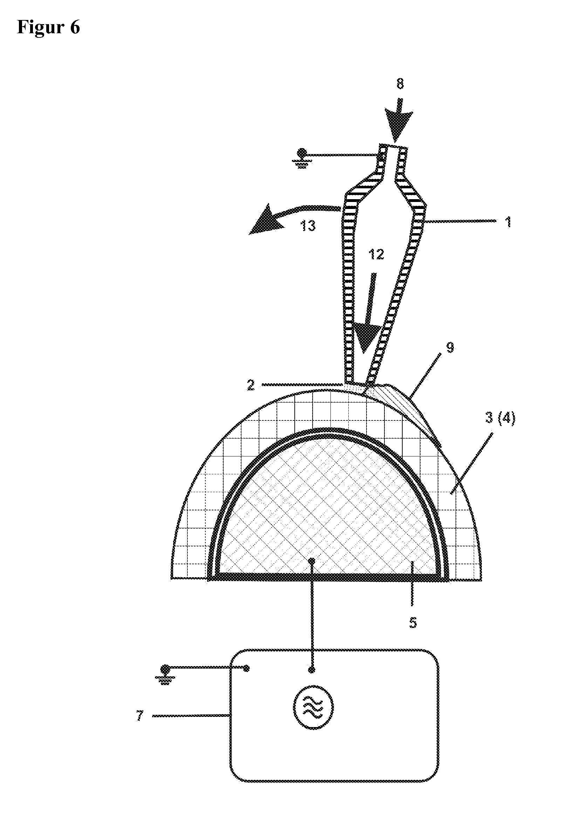

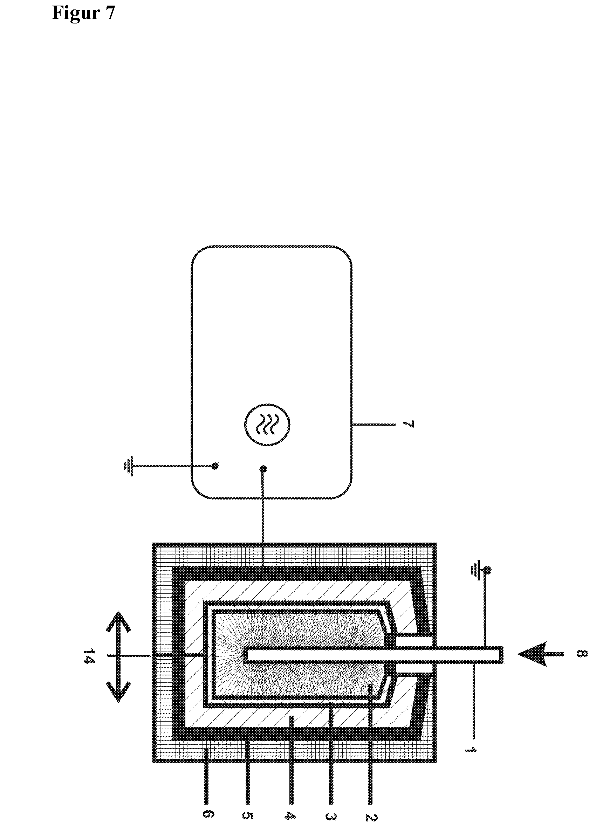

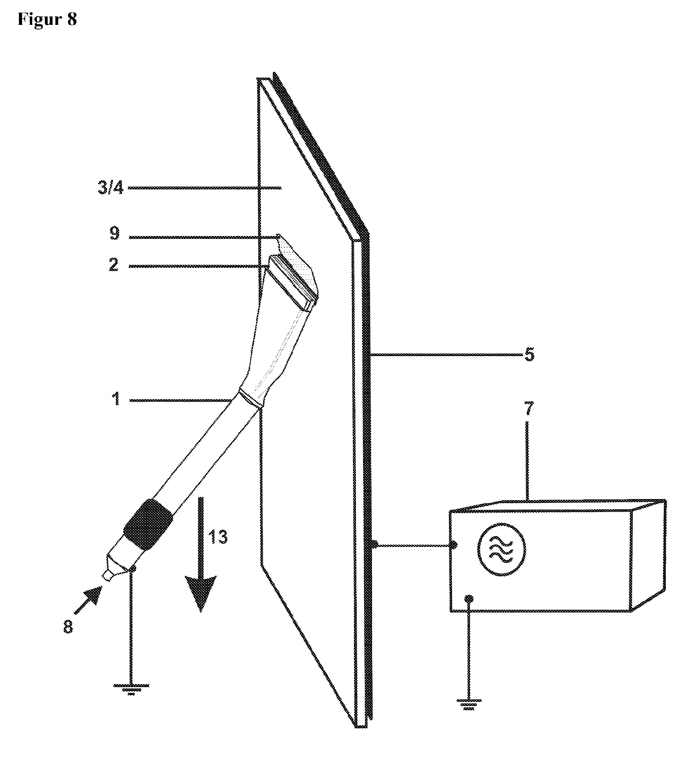

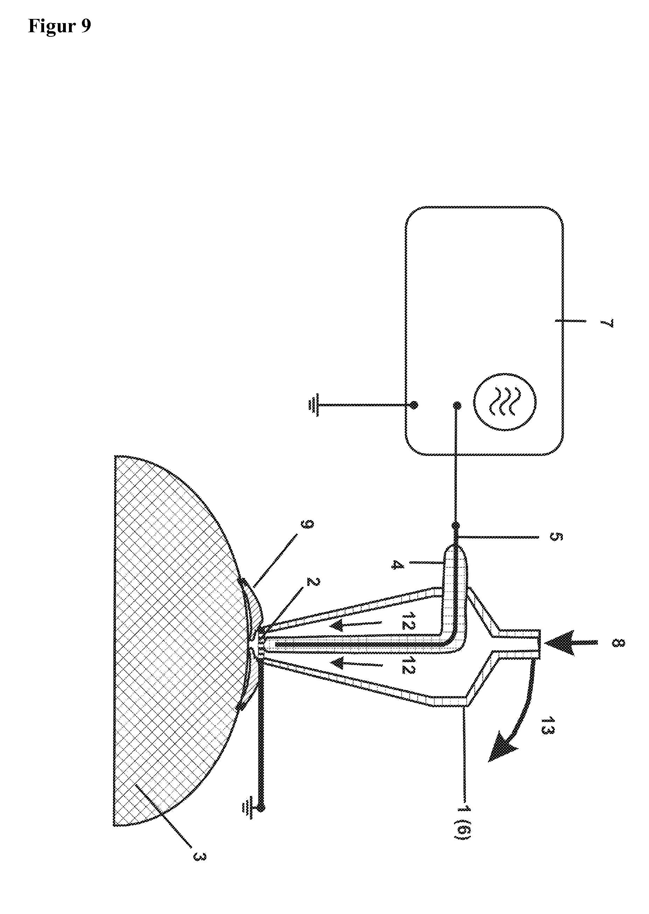

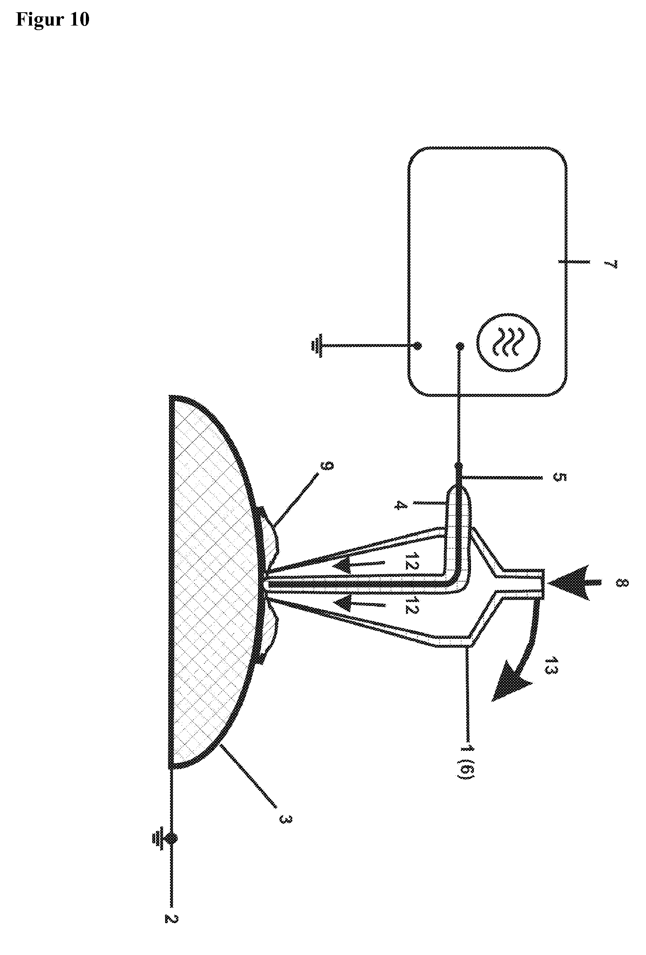

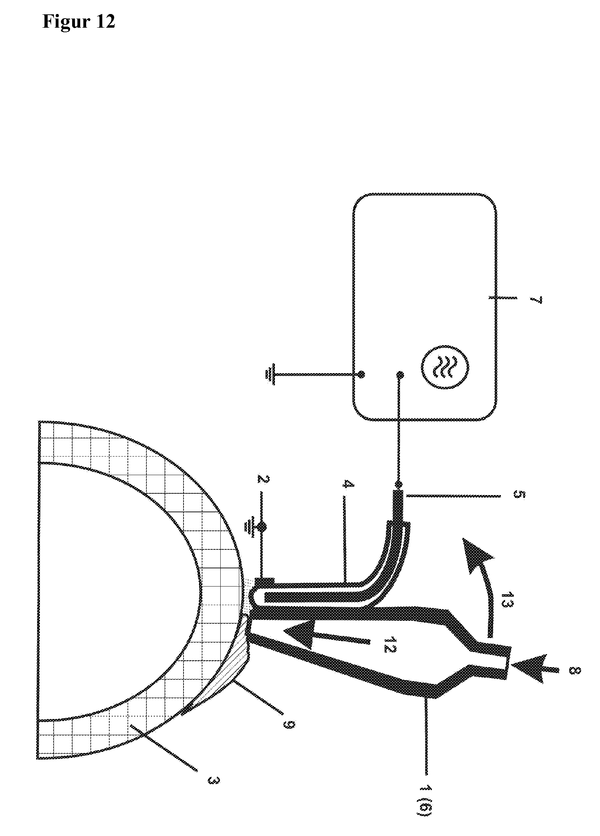

Fig. 1 zeigt den prinzipiellen Aufbau der Vorrichtung mit einer ebenen, mit einem Dielektrikum- oder Ferroelektrikum (4) bedeckten Hochspannungselektrode (5) sowie mit einer, als geerdete Kontaktelektrode ausgebildeten, Gasdüse (1), die auf einem auf dem Dielektrikum (4) aufliegenden Werkstück (3) gleitend ein Oberflächenplasma (9) erzeugt. Die Gaszufuhr (8) erfolgt über den Gasanschluss der Breitstrahl-Gasdüse (1). Die geerdete Kontaktelektrode (2) kann dabei entweder als Gleitkontakt aus elektrisch leitendem Elastomer, wie in Fig. 2 dargestellt, als elektrisch leitende Rolle, wie in Fig. 3 gezeigt, oder als Pinsel aus elektrisch leitenden Borsten, wie in Fig. 4 demonstriert, ausgeführt werden. Fig. 5 zeigt den gleichen Aufbau wie Fig. 1, bei dem jedoch ein Metallrohr mit einem schmalen Schlitz für den Gasaustritt gleichzeitig als Breitstrahl-Gasdüse (1) und als geerdete Kontaktelektrode (2) fungiert. Fig. 6 zeigt eine Anordnung zur Behandlung von Hohlkörpern aus Kunststoff (3), bei der eine elektrisch leitende Füllung des Hohlkörpers als Hochspannungselektrode dient und der Hohlkörper gleichzeitig als Dielektrikum (4) der Hochspannungselektrode wirkt. In Fig. 7 ist der prinzipielle Aufbau zur Innenbehandlung (trockene Reinigung, Desinfektion und/oder Beschichtung) von Flaschen dargestellt. Fig.8 zeigt als Anwendungsbeispiel eine Anordnung zur trockenen Reinigung und Desinfektion von speziell konstruierten Tischen und Wandbelägen in hygienisch sensiblen Bereichen, wie zum Beispiel in Operationssälen bzw. im Bereich der Lebensmittelverarbeitung. Die Beläge der Tische und Wände sind dafür so zu gestalten, dass sie als mit einem Isolator (4) bedeckte Hochspannungselektrode (5) geschaltet werden können. Fig. 9 demonstriert den prinzipiellen Aufbau eines kompakten Handgerätes zur Behandlung komplex geformter Kunststoff-Oberflächen, bei dem die mit einem Isolator (4) bedeckte Hochspannungselektrode (5) in der Gasdüse (1) angeordnet ist. Am Ende dieser Hochspannungselektrode ist in der Ebene der Düsenöffnung die elektrisch leitende, als Sieb gestaltete, geerdete Kontaktelektrode (2) angeordnet. Die in Fig. 10 gezeigte Anordnung ist ähnlich konstruiert. Diese Vorrichtung ist zur Behandlung von komplex geformten Metalloberflächen vorgesehen. Da in diesem Fall die Metalloberfläche selbst als leitende, geerdete Kontaktelektrode dient, kann auf eine, an der Düse montierte, spezielle Kontaktelektrode verzichtet werden. Wie in Fig. 12 dargestellt, kann in einer ähnlichen Vorrichtung die mit einem Dielektrikum (4) umgebene Hochspannungselektrode (5) auch außerhalb der Gasdüse angeordnet werden. Die in den Fig. 10 und 12 gezeigten Anordnungen sind aber nicht Teil der beanspruchten Erfindung.

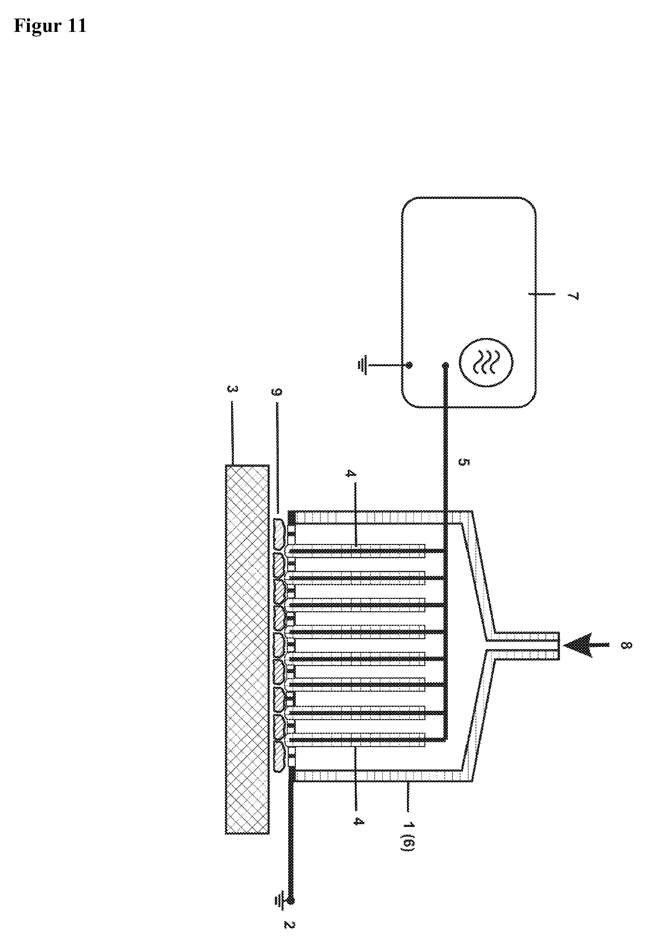

Fig. 11 zeigt eine weitere Ausführungsform eines kompakten Handgerätes, bei der anstelle einer einzelnen isolatorbedeckten Hochspannungselektrode ein Array aus mehreren Einzelelektroden verwendet wird. Auf diese Weise werden Oberflächenentladungen größerer Flächenausdehnung erzeugt, so dass sich die notwendigen Behandlungszeiten entsprechend verkürzen lassen.

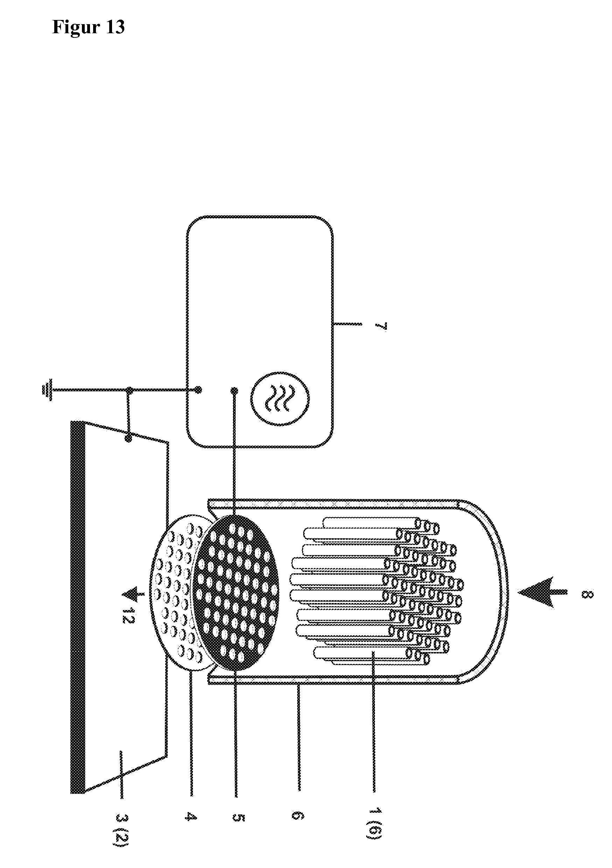

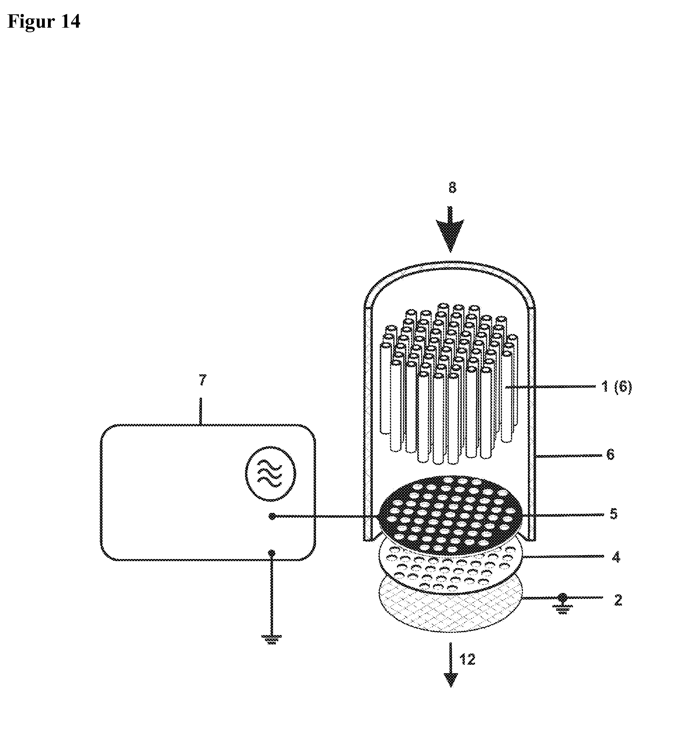

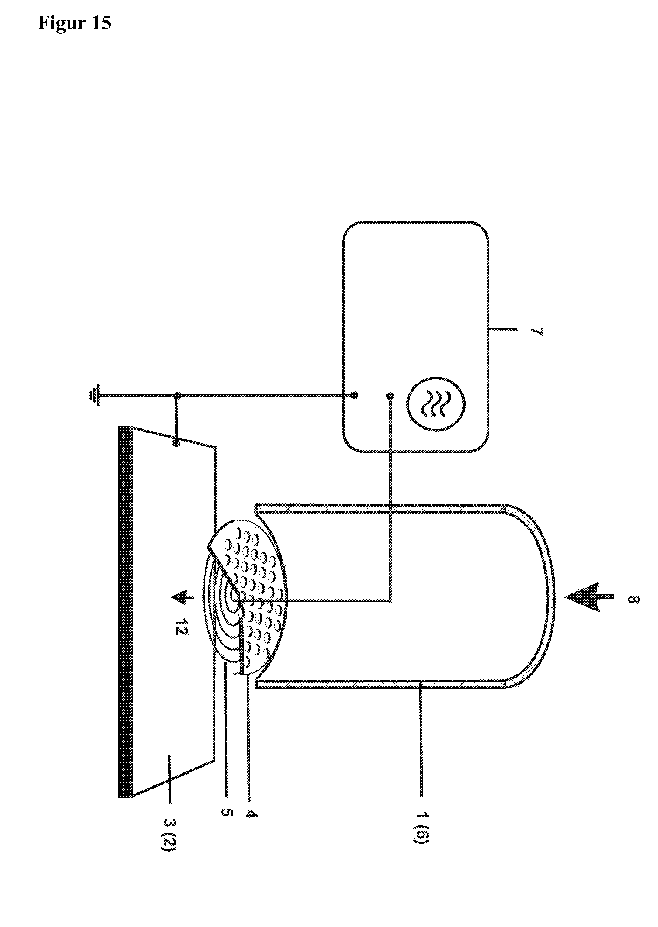

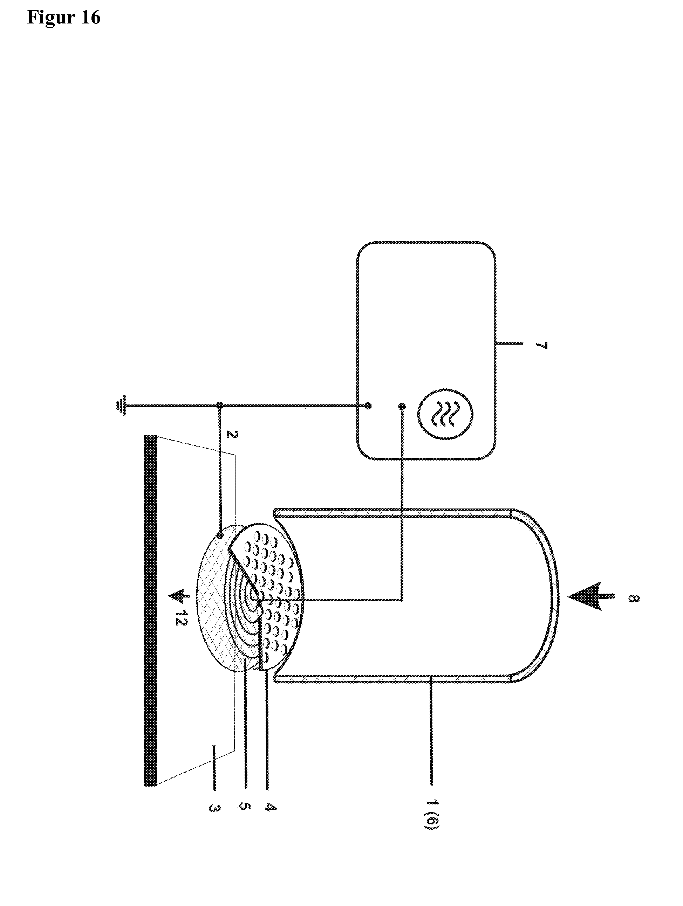

Weitere Konstruktionsbeispiele für kompakte Handgeräte sind in Fig. 13 bis Fig. 16 dargestellt. Bei der Anordnung in Fig. 13 handelt es sich dabei um eine als kompakte Mehrkanal-Plasmadüse gestaltete Ausführungsform zur Behandlung von Metalloberflächen, bei der das metallische Werkstück als geerdete Kontaktelektrode fungiert und bei Fig. 14 um eine gleich geartete Anordnung für die Behandlung von Kunststoff-Oberflächen mit einer, in der Ebene der Düsenöffnungen angeordneten, Metall-Gaze als geerdete Kontaktelektrode (2). Bei den in Fig. 15 und Fig. 16 dargestellten Lösungen strömt das Prozessgas durch eine Lochplatte aus Isoliermaterial (6), vor der spiralförmig die isolatorbedeckte Hochspannungselektrode (4/5) angeordnet ist. Fig. 15 stellt dabei, in ähnlicher Weise, wie in Fig. 13, den Fall für die Behandlung von Metall-Oberflächen (als geerdete Kontaktelektrode wirkend) und Fig. 16, ähnlich wie in Fig. 14, den Fall für die Behandlung von Kunststoff-Oberflächen (Metall-Gaze als geerdete Kontaktelektrode) dar. Die in den Abb. 13 und 15 gezeigten Anordnungen sind aber nicht Teil der beanspruchten Erfindung.

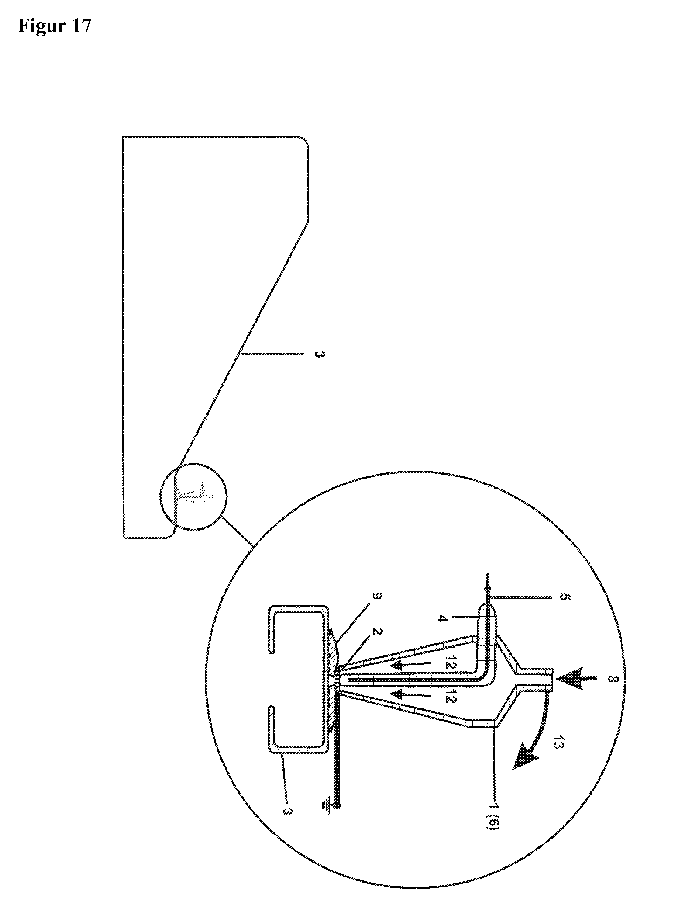

In Fig. 17 wird die Möglichkeit der Anwendung eines kompakten Handgerätes, wie es in Fig. 9 dargestellt ist, zur trockenen Reinigung beziehungsweise Desinfektion von Handläufen (3) (beispielsweise bei Rolltreppen) demonstriert.

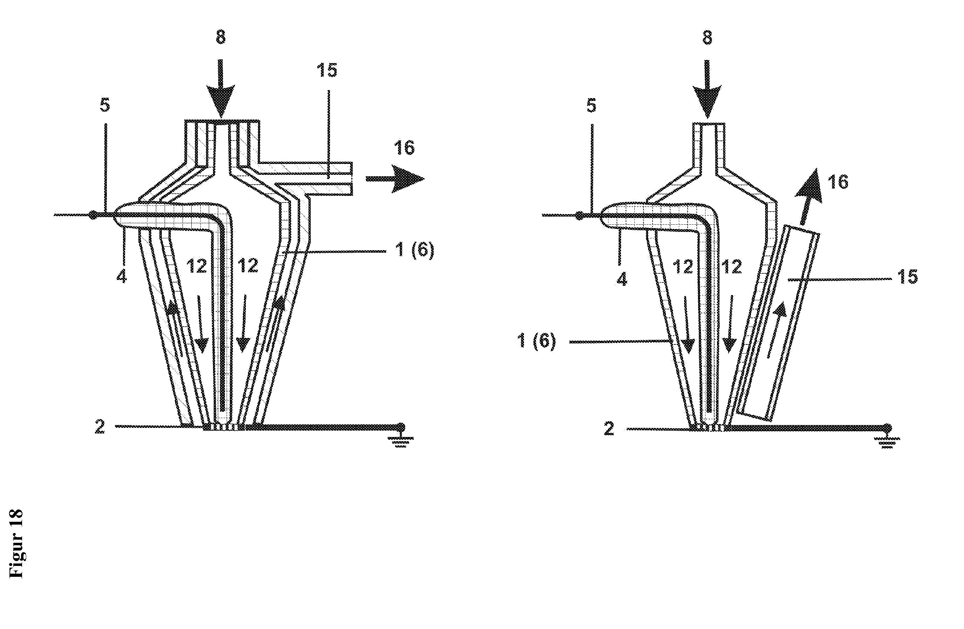

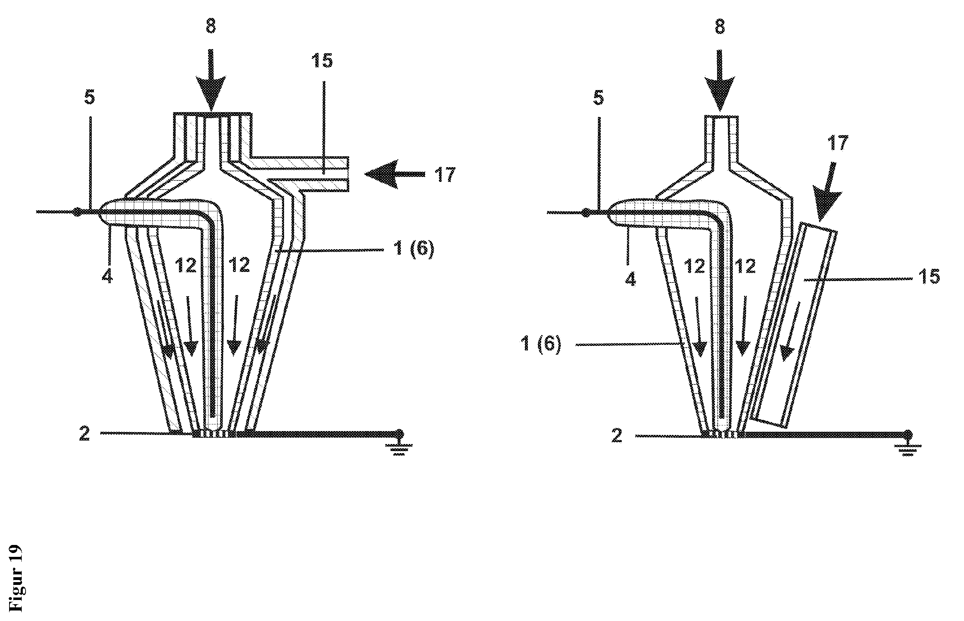

Fig. 18 zeigt zwei Möglichkeiten der Anordnung eines zweiten Düsenkanals (15) zur Absaugung (16) von, durch die Entladung produziertem, Ozon. Diese Düsenkanäle (15) können, wie in Fig. 19 dargestellt, ebenso für die Precursor-Zufuhr (17) für die beschichtende Behandlung verwendet werden.

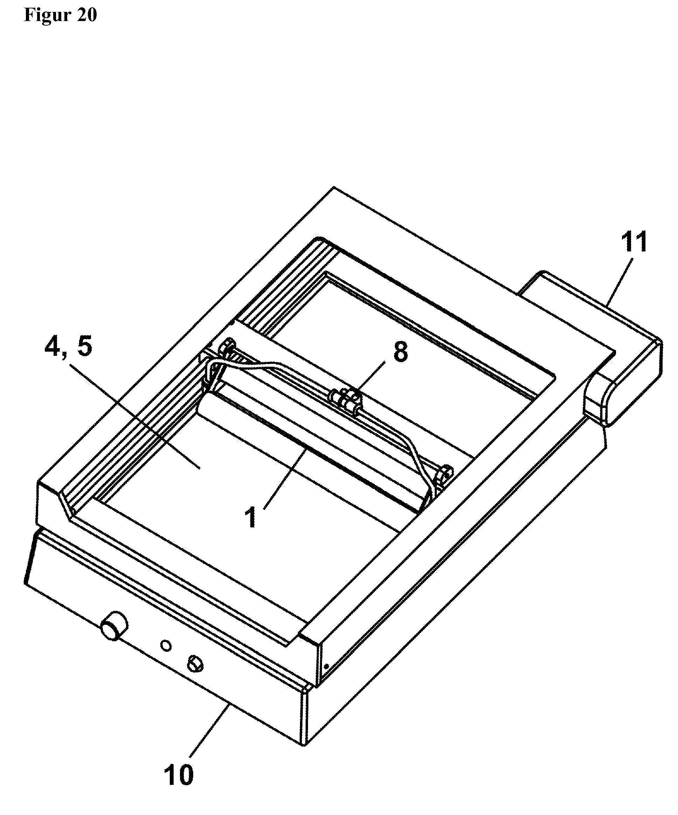

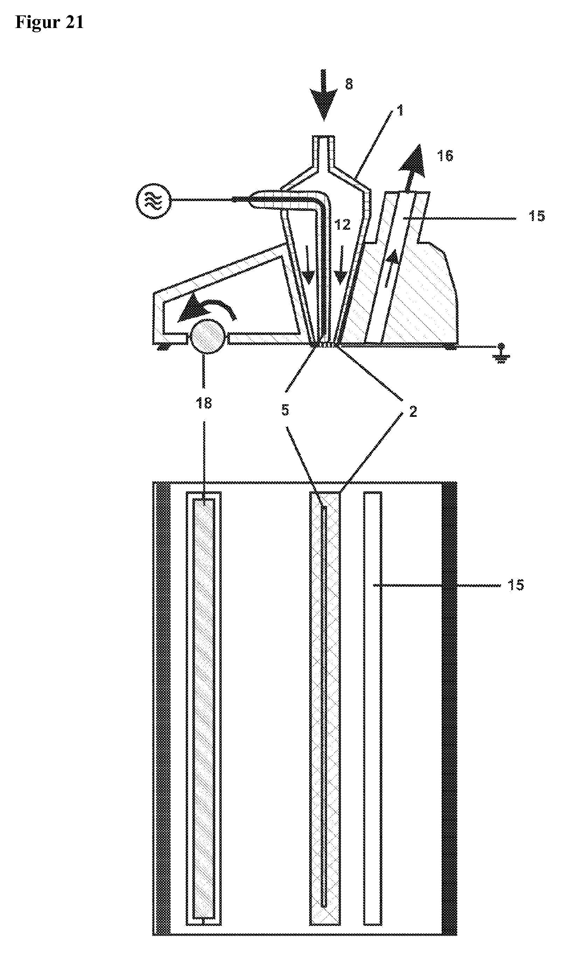

In Fig. 20 ist ein nach dem in Fig. 1 bis Fig. 5 erläuterten Prinzip arbeitendes, motorgesteuertes Tischgerät dargestellt. Als ein weiteres Anwendungsbeispiel ist in Fig. 21 eine Vorrichtung zur kombinierten Reinigung mittels einer rotierenden Bürste (18), zur trockenen Reinigung und Desinfektion mittels Plasmabehandlung durch eine Anordnung, wie sie in Fig. 9 dargestellt ist, sowie zur Absaugung (16) von Staub und Ozon über eine weitere Düse (15) dargestellt.

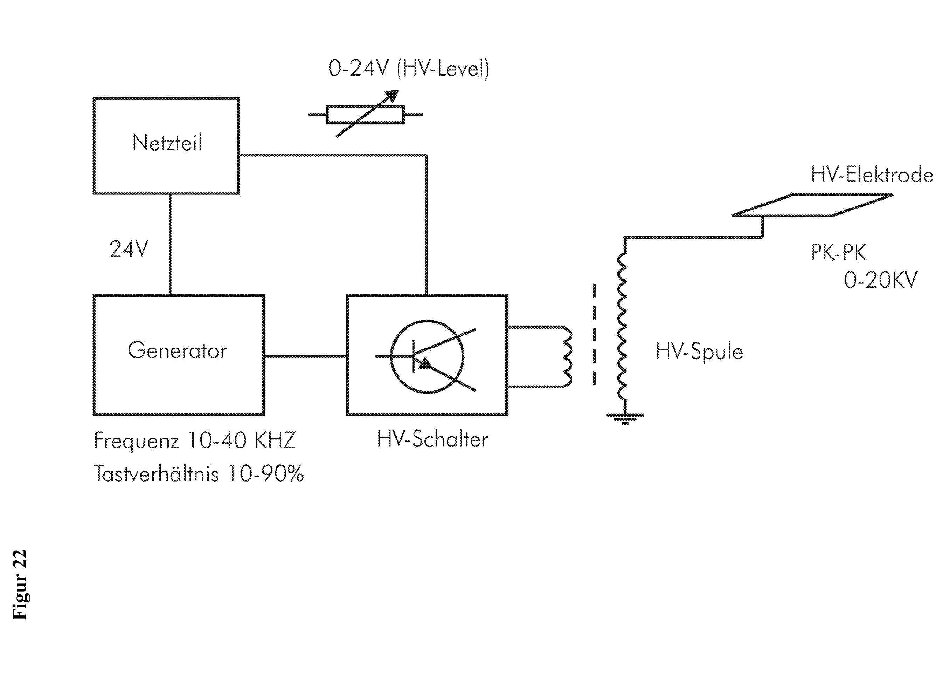

Fig. 22 zeigt das Prinzipschaltbild der Spannungsversorgung.

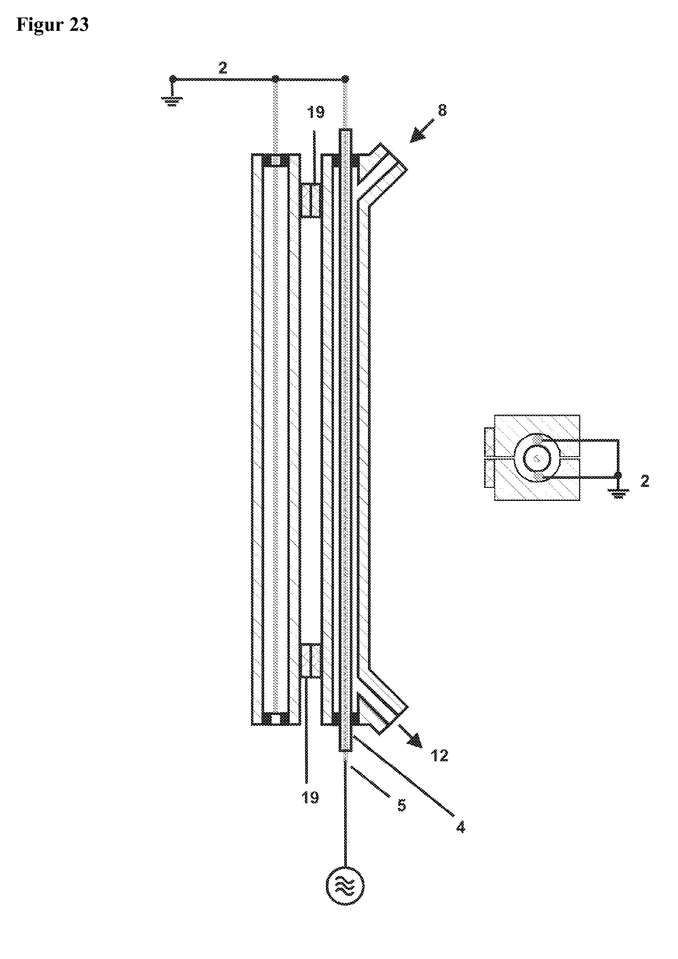

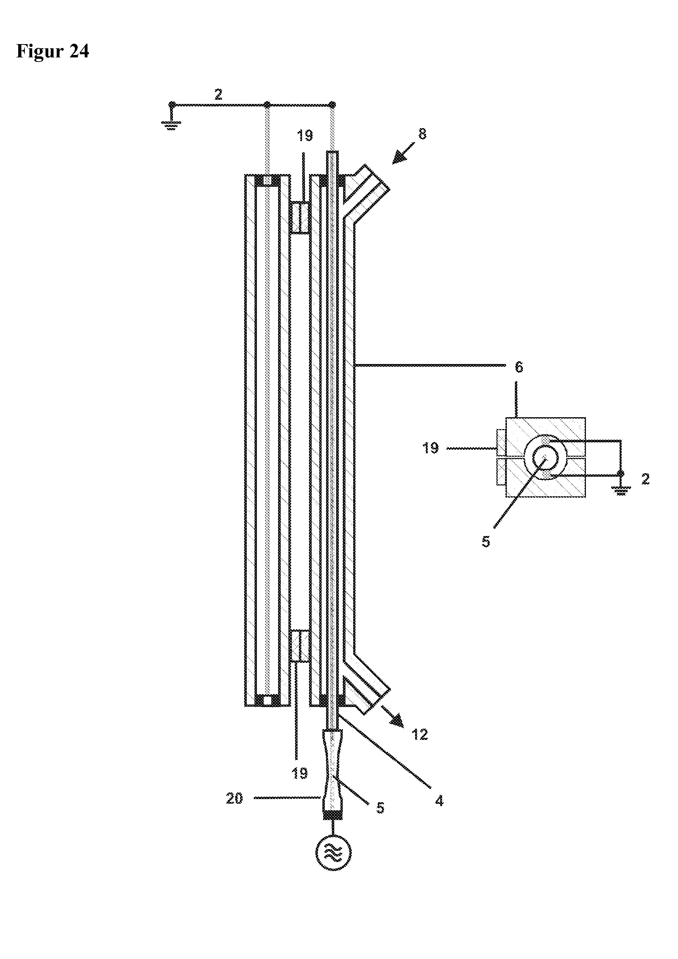

In Fig. 23 wird eine auf dem Arbeitsprinzip der Erfindung basierende Behandlungseinheit für die äußere Behandlung von isolierten Drähten zur Verbesserung der Benetzbarkeit, sowie in Fig. 24 eine ähnlich aufgebaute Behandlungseinheit zur trockenen Reinigung und Entkeimung der äußeren Oberfläche von Kathetern dargestellt. In beiden Fällen wirken die zu behandelnden Objekte als eine, mit einem Dielektrikum (4) bedeckte, Hochspannungselektrode (5) und die elektrisch leitende, geerdete Kontaktelektrode (2) besteht aus zwei, eng an den Objekten anliegenden, dünnen Drähten. Ein aus zwei, mit Scharnieren (19) verbundenen, aufklappbaren, Hälften bestehendes Rohr aus Isoliermaterial ermöglicht sowohl die Halterung und definierte Positionierung der Objekte, als auch die fein dosierte Gaszufuhr (8).

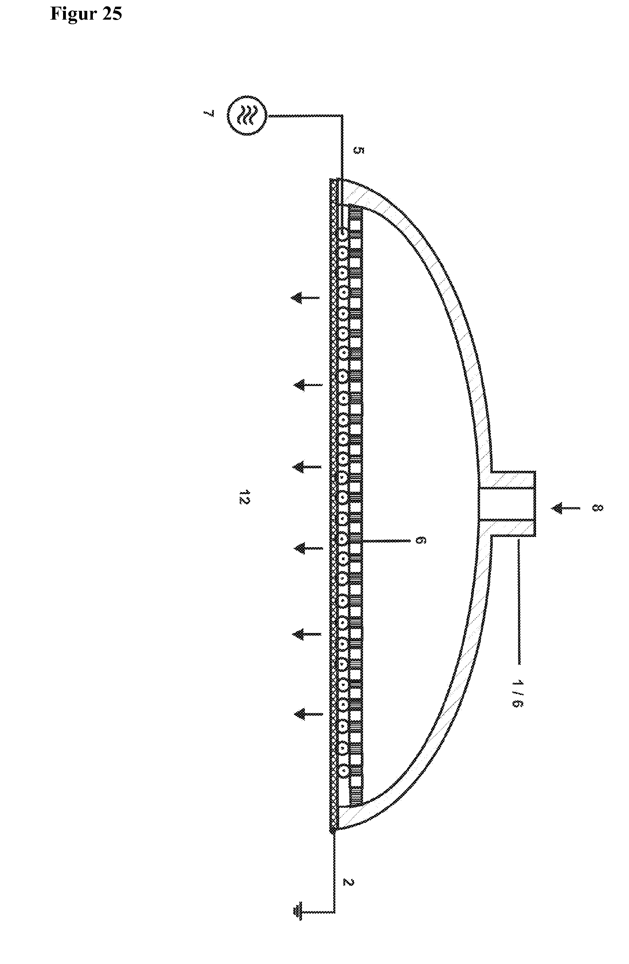

Fig. 25 zeigt eine spezielle Ausführungsform der in Fig. 16 dargestellten Anordnung. In diesem Fall hat die Gasdüse eine flachere Form und ist, ebenso wie die Elektroden und die gasdurchlässige Isolierschicht in der Ebene des Gasaustritts aus elastischen Materialien hergestellt. Diese Anordnung gestattet, die Kontaktfläche an verschiedene Körperoberflächen anzuschmiegen und ist damit prinzipiell geeignet, in engem Hautkontakt in der Art einer elastischen Manschette auf Bereiche des menschlichen Körpers aufgelegt zu werden, um damit gegebenenfalls erkrankte Hautpartien durch die erzeugte Oberflächenentladung behandeln zu können.

## Patentansprüche

1. Vorrichtung zur trockenen Reinigung, Aktivierung, Beschichtung, Modifikation und biologischen Dekontamination einer Oberfläche (3) mittels eines, durch eine Oberflächen-Barrierenentladung in einer definierten, strömenden Gasatmosphäre erzeugten Atmosphärendruckplasmas, umfassend eine mit einem Dielektrikum oder Ferroelektrikum (4) bedeckte Hochspannungselektrode (5) und eine elektrisch leitende, geerdete Kontaktelektrode (2), die dazu ausgelegt ist, im Betrieb eine Kontaktstelle mit der mit einem Dielektrikum oder Ferroelektrikum bedeckten Hochspannungselektrode aufzuweisen, weiter umfassend eine Hochspannunsversorgung (7), eine Gaszufuhr (8) und eine Gasdüse (1) mit einer Gasaustrittsöffnung (12), **dadurch gekennzeichnet, dass**
a) die Gasdüse (1) in die Kontaktelektrode (2) integriert ist oder
b) die Gasdüse (1) selbst als geerdete Kontaktelektrode (2) fungiert, und weiter **dadurch gekennzeichnet, dass** die Gasaustrittsöffnung (12) so konstruiert ist, dass im Betrieb ein austretender Gasstrom auf die Kontaktstelle zwischen der mit einem Dielektrikum bedeckten Hochspannungselektrode (5) und der geerdeten Kontaktelektrode (2) gerichtet ist.

2. Vorrichtung nach Anspruch 1, **dadurch gekennzeichnet, dass** sie zusätzlich mindestens eines der folgenden Elemente enthält: Isolation (6), ein Gehäuse für die Hochspannungsversorgung (10), einen Motor, vorzugsweise mit Magnetkupplung (11), Fügekante (14), zweiter Düsenkanal (15), Absaugung (16), Precursor-Zufuhr (17), Scharnier (19) oder Griffstück mit Steckeranschluss (20).

3. Vorrichtung nach Anspruch 1 oder 2, **dadurch gekennzeichnet, dass** als Gasdüse (1) eine Breitstrahldüse fungiert, deren Schlitz vorzugsweise 0,2 - 0,3 mm beträgt.

4. Vorrichtung nach einem der Ansprüche 1 bis 3 **dadurch gekennzeichnet, dass** als Spannungsquelle (7) ein Handgerät dient.

5. Vorrichtung nach einem der Ansprüche 1 bis 4, **dadurch gekennzeichnet, dass** die elektrisch leitende geerdete Kontaktelektrode (2)
a) als Gleitkontakt oder als kleine Walze, Rolle, Bürste oder Pinsel ausgeführt ist und / oder
b) aus Metall oder anderen elektrisch leitenden Materialien, vorzugsweise elektrisch leitenden Elastomeren, besteht.

6. Vorrichtung nach einem der Ansprüche 1 bis 5, **dadurch gekennzeichnet, dass**
a) die zu behandelnde Oberfläche (3) als dielektrikumbedeckte Hochspannungselektrode (5) genutzt wird oder
b) die dielektrikumbedeckte Hochspannungselektrode (5) als ebene Form oder als rotierende Walze ausgestaltet ist oder
c) die Oberfläche (3) mit einer elektrisch leitenden Form umschlossen ist und diese Form die dielektrikumbedeckte Hochspannungselektrode (5) bildet.

7. Vorrichtung nach einem der Ansprüche 1 bis 6, **dadurch gekennzeichnet, dass** sie ein kompaktes Handgerät darstellt, in dem die eine oder mehrere dielektrikum-bedeckte Hochspannungselektroden gemeinsam mit einer aus Metall-Gaze oder einem gelochten Blech gebildeten, geerdeten Kontaktelektrode in dem Handgerät integriert sind und in der Ebene des Gasaustritts aus der Gasdüse angeordnet sind, so dass in diesem Bereich auf der Oberfläche des Dielektrikums der Hochspannungselektroden eine intensivierte Oberflächenentladung erzeugt wird.

8. Tischgerät für die Behandlung von ebenen Materialien mit einer eingeschränkten Flächenausdehnung, das die Vorrichtung gemäß einem der Ansprüche 1 bis 7 enthält und vorzugsweise dazu ausgelegt ist, dass im Betrieb eine Abtastung der Fläche, ähnlich wie bei einem optischen Scanner, über einen Motorantrieb erfolgt.

9. Verfahren zur trockenen Reinigung, Aktivierung, Beschichtung und biologischen Dekontamination einer Oberfläche mittels der Vorrichtung gemäß einem der Ansprüche 1 bis 8, **gekennzeichnet durch** folgende Schritte:
a) ein zu behandelndes Material (3) befindet sich entweder zwischen der mit einem Dielektrikum oder Ferroelektrikum (4) bedeckten Hochspannungselektrode (5) und der geerdeten Kontaktelektrode (2) oder an deren Kontaktstelle
b) ein Prozessgasstrom wird aus der Gasdüse (1) auf die Kontaktstelle der geerdeten Kontaktelektrode (2) gerichtet
c) gleichzeitig oder unmittelbar danach wird eine Spannung an die Hochspannungselektrode (5) angelegt und
d) die Kontaktelektrode (2) mit der Gasdüse (1) und das zu behandelnde Material (3) werden relativ zueinander bewegt,
wobei auf der Oberfläche des zu behandelnden Materials (3), an der sich die Kontaktelektrode (2) mit der Gasdüse (1) befindet, in der Prozessgasströmung eine Oberflächen-Barrierenentladung erzeugt wird.

10. Verfahren nach Anspruch 9, **dadurch gekennzeichnet, dass** als Prozessgas ein Edelgas, vorzugsweise Argon, in reiner Form oder als Gemisch mit anderen Gasen, eingesetzt wird.

11. Verfahren nach Anspruch 9 oder 10, **dadurch gekennzeichnet, dass**
a) das zu behandelnde Material (3) auf einer Isolierstoffschicht der ebenen, dielektrikum- oder ferroelektrikumbedeckten Hochspannungselektrode (5) aufgelegt wird und die elektrisch leitende, geerdete, mit einer Breitstrahl-Gasdüse (1) gekoppelte Kontaktelektrode (2) gleitend über die zu behandelnde Kunststoffoberfläche geführt wird und / oder
b) für die Behandlung von ebenen Materialien mit einer eingeschränkten Flächenausdehnung, z.B. in den Formaten DIN A6 bis DIN AO, bei der die Abtastung der Fläche, ähnlich wie bei einem optischen Scanner, über einen Motorantrieb erfolgt und / oder
c) zur Behandlung von längeren Bahnmaterialien oder Platten, bei der anstelle einer ebenen eine als rotierende Walze ausgestaltete, dielektrikum- oder ferroelektrikumbedeckte Hochspannungselektrode verwendet wird und mittels einer geeigneten Vorschubeinrichtung das zu behandelnde Material zwischen der rotierenden Hochspannungselektrode und der auf der Oberfläche des Materials gleitenden oder rollenden, als geerdete Kontaktelektrode gestalteten, Breitstrahl-Gasdüse hindurch bewegt wird.

12. Verfahren nach Anspruch 11, **dadurch gekennzeichnet, dass**
a) Hohlkörper aus Kunststoff behandelt werden, indem die Hohlkörper mit einer leitfähigen Masse, beispielsweise mit Stahlwolle, leitfähigem plastischen Material oder mit elektrisch leitender Flüssigkeit, gefüllt werden, die mit der Hochspannung verbunden wird und so gemeinsam mit dem Kunststoff-Hohlkörper als dielektrikumbedeckte Hochspannungselektrode wirkt oder
b) zur trockenen Reinigung beziehungsweise biologischen Dekontamination der inneren Oberfläche von Flaschen, die Flasche passgenau von zwei Hälften einer elektrisch leitenden, auf Hochspannungspotential gelegten, Form umschlossen wird, die zusammen mit der Flaschenwand als dielektrikumbedeckte Hochspannungselektrode wirkt und im Inneren der Flasche eine Flaschenbürste aus elektrisch leitendem Material mit eng an der inneren Oberfläche der Flasche anliegenden Borsten angeordnet ist, die gleichzeitig als geerdete Kontaktelektrode wirkt und mit einer Gasdüse gekoppelt ist.

13. Verfahren nach Anspruch 11, **dadurch gekennzeichnet, dass** für die äußere Behandlung von isolierten Drähten zur Verbesserung der Benetzbarkeit die zu behandelnden Drähte als eine dielektrikumbedeckte Hochspannungselektrode genutzt werden und zusammen mit den eng an dem zu behandelnden Draht anliegenden, elektrisch leitenden, geerdeten Kontaktelektroden, bestehend aus zwei dünnen Drähten, in einem Rohr aus Isoliermaterial angeordnet sind.

14. Verfahren nach einem der Ansprüche 9 bis 13, **dadurch gekennzeichnet, dass** zur Beschichtung der Oberfläche dem Prozessgas direkt oder über eine zweite Düse ein Precursor, vorzugsweise aus siliziumorganischen Verbindungen, wie HMDSO oder TEOS, zur Erzeugung von SiOx- Schichten, zugemischt wird.

15. Verfahren nach einem der Ansprüche 9 oder 14, **dadurch gekennzeichnet, dass** als Hochspannung eine kontinuierliche oder gepulste Wechselspannung, gepulste Gleichspannung oder einzelne Hochspannungspulse verwendet werden, vorzugsweise eine Wechselspannung als Sinus-, Rechteck- oder Dreiecksfunktion.

16. Verwendung der Vorrichtung gemäß den Ansprüchen 1 bis 8 oder des Verfahrens gemäß den Ansprüchen 9 bis 15 zur
a) Behandlung von Oberflächen in Operationssälen oder
b) Behandlung von Oberflächen im Lebensmittelbereich oder
c) Behandlung von Metalloberflächen oder
d) Behandlung von Kunststoffoberflächen oder
e) Reinigung und Desinfektion von Treppen-Handläufen oder
f) Beseitigung von Trennmittel-Rückständen auf Oberflächen oder
g) äußeren Behandlung von isolierten Drähten oder
h) trockenen Reinigung, Desinfektion und biologischen Dekontamination von in medizinischen Geräten oder Instrumenten verwendeten Schläuchen oder
i) Behandlung oder Beschichtung der inneren Oberflächen von Rohren oder Schläuchen oder
j) Plasmabehandlung von Folien oder Schildern aus Kunststoff, insbesondere zur Verbesserung der Haftung von Klebefolien oder Druckfarben auf diesen Materialien.

## Claims

1. Device for dry cleaning, activation, coating, modification and biological decontamination of surfaces (3) by means of an atmospheric pressure plasma generated by a surface barrier discharge in a defined flowing gas atmosphere comprising a high voltage electrode (5) covered with a dielectric or ferroelectric (4) and an electrically conductive, grounded contact electrode (2) which is designed in operation to comprise a contact point with the high voltage electrode covered with a dielectric or a ferroelectric, comprising further a high voltage supply (7) and a gas supply (8), a gas nozzle (1) with a gas outlet opening (12), **characterised in that**
a) the gas nozzle (1) is integrated into the contact electrode (2) or
b) the gas nozzle (1) itself acts as a grounded contact electrode (2) and
the gas outlet opening (12) is designed such that an escaping gas flow is directed towards the contact point between the high voltage electrode covered with a dieelectric and the grounded contact electrode (2).

2. Device according to claim 1, **characterised in that** in addition it contains at least one of the following elements: isolation (6), a housing for the high voltage supply (10), a motor, preferably with magnetic clutch (11), joint edge (14), second nozzle channel (15), suction (16), precursor supply (17), hinge (19) or handle piece with connector (20).

3. Device according to claim 1 or 2, **characterised in that** a wide angle nozzle acts as a gas nozzle (1) the slot of which is preferably 0.2 - 0.3 mm.

4. Device according to any one of claims 1 to 3, **characterised in that** a handset serves as a voltage source (7).

5. Device according to any one of claims 1 to 4, **characterised in that** the electrically conductive, grounded contact electrode (2)
a) is carried out as a sliding contact or as a small cylinder, roll, scrubber or brush and / or
b) is comprised of metal or of other electrically conductive materials, preferably electrically conductive elastomers.

6. Device according to any one of claims 1 to 5, **characterised in that** the
a) the surface to be treated (3) is used as a dielectric covered high voltage electrode (5) or
b) the dielectric covered high voltage electrode (5) is designed as an even shape or as a rotating cylinder or
c) the surface (3) is enclosed with an electrically conductive shape and said shape forms the dielectric covered high voltage electrode (5).

7. Device according to any one of claims 1 to 6, **characterised in that** it constitutes a compact handset in which the one or several dielectric covered high voltage electrodes are jointly integrated with a grounded contact electrode formed from metal gauze or a perforated plate into the handset, and are located in the plane of the gas outlet from the gas nozzle so that in this area on the surface of the dielectric of the high voltage electrodes an intensified surface discharge is generated.

8. Tabletop device for treatment of flat materials with a restricted surface area which contains the device according to any one of claims 1 to 7, and is preferably designed for a scanning of the surface occurring during operation similar to an optical scanner via a motor drive.

9. Method for dry cleaning, activation, coating, and biological decontamination of a surface by means of the device according to any one of claims 1 to 8, **characterised by** the following steps:
a) a material to be treated (3) is either between the high voltage electrode (5) covered with a dielectric or a ferroelectric (4) and the grounded contact electrode (2) or at its contact point
b) a process gas flow is directed from the gas nozzle (1) to the contact point of the grounded contact electrode (2)
c) at the same time or immediately thereafter a voltage is applied to the high voltage electrode (5) and
d) the contact electrode (2) with the gas nozzle (1) and the material to be treated (3) are moved relative to each other,
with a surface barrier discharge being generated in the process gas flow on the surface of the material to be treated (3) where the contact electrode (2) with the gas nozzle (1) is located.

10. Method according to claim 9, **characterised in that** a noble gas is used as a process gas, preferably argon in pure form or as a mixture with other gases.

11. Method according to claim 9 or 10, **characterised in that**
a) the material to be treated (3) is placed on an insulating material layer of the flat high voltage electrode (5) covered with dielectric or ferroelectric, and the electrically conductive, grounded contact electrode (2) coupled with a wide angle gaz nozzle (1) is lead glidingly over the plastic surface to be treated, and/or
b) for treatment of flat materials with a restricted surface area, e.g. in the formats DIN A6 to DIN A0, where scanning of the surface similar to an optical scanner occurs via a motor drive, and/or
c) for treatment of longer web materials or plates where instead of a flat high voltage electrode covered with dielectric or ferroelectric, a high voltage electrode designed as a rotating cylinder will be used, and by means of an appropriate feeding device the material to be treated will be moved between the rotating high voltage electrode and the wide angle gas nozzle sliding or rolling on the surface of the material and designed as a contact electrode.

12. Method according to claim 11, **characterised in that**
a) hollow bodies made from plastic are treated by filling the hollow bodies with a conductive compound, for example with steel wool, conductive plastic material or with electrically conductive liquid, which is connected to the high voltage, and thus acts jointly with the plastic hollow body as a high voltage electrode covered with dielectric, or
b) for dry cleaning and/or biological decontamination of the inner surface of bottles, the bottle is enclosed true to size by two halves of an electrically conductive shape put at high voltage potential, which together with the bottle wall acts as a high voltage electrode covered with dielectric, and inside the bottle a bottle brush out of electrically conductive material with bristles fitting close to the inner bottle surface is located, which at the same time acts as a grounded contact electrode and is coupled with a gaz nozzle.

13. Method according to claim 11, **characterised in that** for external treatment of insulated wires for improvement of wettability the wires to be treated are used as a high voltage electrode covered with dielectric, and together with the electrically conductive, grounded contact electrodes fitting close to the wire to be treated comprising two thin wires are located in a tube of insulating material.

14. Method according to claim 9 to 13, **characterised in that** for coating of the surface a precursor, preferably made from organosilicon compounds such as HMDSO or TEOS, is mixed to the process gas directly or via a second nozzle for generation of SiOₓ layers.

15. Method according to any one of claims 9 or 14, **characterised in that** as a high voltage a continuous or pulsed alternating voltage, pulsed d.c. voltage or individual high voltage pulses are used, preferably an alternating voltage as a sine, square or triangle function.

16. Use of the device according to claims 1 to 8 or of the method according to claims 9 to 15 for
a) treatment of surfaces in operating rooms or
b) treatment of surfaces in the food sector or
c) treatment of metal surfaces or
d) treatment of plastic surfaces or
e) cleaning and disinfection of staircase handrails or
f) removal of release agent residues on surfaces or
g) external treatment of insulated wires or
h) dry cleaning, disinfection and biological decontamination of flexible tubes used in medical devices or instruments or
i) treatment or coating of the internal surfaces of tubes or flexible tubes or
j) plasma treatment of foils or labels out of plastic, in particular for improvement of adhesion of adhesive foils or printing inks on said materials.

## Revendications

1. Dispositif pour le nettoyage à sec, l'activation, le revêtement, la modification et la décontamination biologique des surfaces (3) au moyen d'un plasma à pression atmosphérique produit par une décharge de surface à barrière dans une atmosphère gazeuse définie courante, comportant une électrode à haute tension (5) recouverte d'un diélectrique ou d'un ferroélectrique (4) et une électrode de contact (2) électriquement conductrice et mise à la terre conformée en service à comporter un point de contact avec l'électrode à haute tension recouverte d'un diélectrique ou d'un ferroélectrique, comportant en outre une alimentation haute tension (7) et une alimentation en gaz (8), une buse à gaz (1) avec un orifice de sortie de gaz (12), **caractérisé en ce que**
a) la buse à gaz (1) est intégrée dans l'électrode de contact (2) ou
b) la buse à gaz (1) elle-même agit comme électrode de contact (2) mise à la terre et l'orifice de sortie de gaz (12) est construit de manière qu'un écoulement de gaz sortant est dirigé vers le point de contact entre l'électrode à haute tension recouverte d'un diélectrique et l'électrode de contact (2) mise à la terre.

2. Dispositif selon la revendication 1, **caractérisé en ce qu'**en outre il comprend au moins l'un des éléments suivants: une isolation (6), un carter pour l'alimentation haute tension (10), un moteur, préférablement avec un embrayage magnétique (11), une arête de jointure (14), un deuxième canal de buse (15), une aspiration (16), une alimentation de précurseur (17), une charnière (19) ou une poignée avec connecteur (20).

3. Dispositif selon la revendication 1 ou 2, **caractérisé en ce qu'**une buse de surface large agit comme buse à gaz (1) dont la fente est de préférence 0,2 - 0,3 mm.

4. Dispositif selon l'une quelconque des revendications 1 à 3, **caractérisé en ce qu'**un appareil portatif sert de source de tension.

5. Dispositif selon l'une quelconque des revendications 1 à 4, **caractérisé en ce que** l'électrode de contact électriquement conductrice et mise à la terre (2)
a) est exécuté comme contact de glissement ou comme petit cylindre, rouleau, brosse ou pinceau et/ou
b) comporte du métal ou des autres matériaux électriquement conducteurs, de préférence des élastomères électriquement conducteurs.

6. Dispositif selon l'une quelconque des revendications 1 à 5, **caractérisé en ce que**
a) la surface à traiter (3) est utilisée comme l'électrode à haute tension (5) recouverte d'un diélectrique ou
b) l'électrode à haute tension recouverte (5) d'un diélectrique est conçue comme une forme plane ou un cylindre rotatif ou
c) la surface (3) est entourée d'une forme électriquement conductrice et ladite forme constitue l'électrode à haute tension recouverte d'un diélectrique (5).

7. Dispositif selon l'une quelconque des revendications 1 à 6, **caractérisé en ce qu'**il constitue un appareil portatif compact dans lequel l'une ou les plusieurs électrodes à haute tension recouvertes d'un diélectrique sont intégrées ensemble avec une l'électrode de contact mise à la terre formée de gaze métallique ou d'une tôle perforée dans l'appareil portatif, et sont disposées dans le niveau de la sortie de gaz de la buse à gaz de manière que dans cette zone sur la surface du diélectrique des l'électrodes à haute tension une décharge de surface intensifiée est produit.

8. Appareil de table pour le traitement des matériaux plats avec une extension superficielle limitée comportant le dispositif selon l'une quelconque des revendications 1 à 7 et de préférence est conçu qu'en service un balayage de la surface est effectué comme dans le cas d'un scanner optique par un entraînement par moteur.

9. Procédé pour le nettoyage à sec, l'activation, le revêtement, et la décontamination biologique d'une surface au moyen du dispositif selon l'une quelconque des revendications 1 à 8, **caractérisé par** les étapes suivantes:
a) un matériel à traiter (3) se trouve soit entre l'électrode à haute tension (5) recouverte d'un diélectrique ou d'un ferroélectrique (4) et l'électrode de contact mise à la terre (2) soit à son point de contact
b) un écoulement de gaz de procédé est dirigé de la buse à gaz (1) au point de contact de l'électrode de contact mise à la terre (2)
c) simultanément ou immédiatement après une tension est appliquée à l'électrode à haute tension (5) et
d) l'électrode de contact (2) avec la buse à gaz (1) et le matériel à traiter (3) sont bougées l'une par rapport à l'autre,
une décharge de surface à barrière étant produit dans l'écoulement de gaz de procédé à la surface du matériel à traiter (3) ou se trouve l'électrode de contact (2) avec la buse à gaz (1).

10. Procédé selon la revendication 9, **caractérisé en ce qu'**un gaz rare est utilisé comme gaz de procédé, de préférence argon sous forme pure ou comme mélange avec des autres gaz.

11. Procédé selon la revendication 9 ou 10, **caractérisé en ce que**
a) le matériel à traiter (3) est placé sur une couche d'isolant de l'électrode à haute tension plane recouverte d'un diélectrique ou d'un ferroélectrique (5) et l'électrode de contact électriquement conductrice et mise à la terre (2) liée à une buse de surface large est dirigée de manière glissante sur la surface plastique à traiter et/ou
b) pour le traitement des matériaux plans avec une extension superficielle limitée, par exemple, dans les formats DIN A6 à DIN A0, pour lesquels le balayage de la surface est effectué comme pour un scanner optique par un entraînement par moteur et/ou
c) pour le traitement des matériaux de panneaux ou des plaques pour lesquels, au lieu d'une l'électrode à haute tension plane recouverte d'un diélectrique ou d'un ferroélectrique, une l'électrode à haute tension recouverte d'un diélectrique ou d'un ferroélectrique conçue comme un cylindre rotatif est utilisée, et au moyen d'un dispositif d'avance approprié le matériel à traiter est bougé entre l'électrode à haute tension rotatif et la buse à gaz de surface large glissante ou roulante sur la surface du matériel et conçue comme électrode de contact mise à la terre.

12. Procédé selon la revendication 11, **caractérisé en ce que**
a) des corps creux en plastique sont traités en remplissant les corps creux avec une pâte conductrice, par exemple, avec de la paille de fer, du matériau plastique conducteur ou avec un liquide électriquement conducteur qui est lié avec la haute tension, et agit ainsi ensemble avec le corps creux en plastique comme électrode à haute tension recouverte d'un diélectrique ou
b) pour le nettoyage à sec et/ou la décontamination biologique de la surface interne des bouteilles, la bouteille est entourée sur mesure par deux moitiés d'une forme électriquement conductrice placée sur un potentiel à haute tension qui ensemble avec la paroi de la bouteille agit comme l'électrode à haute tension recouverte d'un diélectrique, et à l'intérieur de la bouteille une brosse de bouteille d'un matériau électriquement conducteur avec des poils en application étroite à la surface interne de la bouteille est disposée qui simultanément agit comme l'électrode de contact mise à la terre et est liée avec une buse à gaz.

13. Procédé selon la revendication 11, **caractérisé en ce que** pour le traitement externe des fils isolés pour l'amélioration de la mouillabilité les fils à traiter sont utilisés comme une l'électrode à haute tension recouverte d'un diélectrique, et ensemble avec les électrodes de contact électriquement conducteurs et mises à la terre en application étroite au fil à traiter comportant deux fils minces sont disposés dans un tube en matériel isolant.

14. Procédé selon l'une quelconque des revendications 9 à 13, **caractérisé en ce que** pour le revêtement de la surface un précurseur, de préférence des composés organosiliciques comme HMDSO ou TEOS, est mélangé au gaz de procédé directement ou par une deuxième buse pour la production des couches SiOₓ.

15. Procédé selon l'une quelconque des revendications 9 ou 14, **caractérisé en ce que** comme haute tension une tension alternative continue ou pulsée, une tension continue pulsée ou des impulsions individuelles de haute tension sont utilisées, de préférence une tension alternative comme fonction sinus, fonction rectangulaire ou triangulaire.

16. Utilisation du dispositif selon les revendications 1 à 8 ou du procédé selon les revendications 9 à 15 pour
a) le traitement des surfaces dans des salles d'opération ou
b) le traitement des surfaces dans le domaine alimentaire ou
c) le traitement des surfaces métalliques ou
d) le traitement des surfaces plastiques ou
e) le nettoyage ou la désinfection des mains courantes d'escaliers ou
f) l'élimination des résidus des agents de séparation sur des surfaces ou
g) le traitement externe des fils isolés ou
h) le nettoyage à sec, la désinfection et la décontamination biologique des tuyaux flexibles utilisés dans des dispositifs ou instruments médicaux ou
i) le traitement ou le revêtement des surfaces internes des tubes ou des tuyaux flexibles ou
j) le traitement par plasma des films ou plaques plastiques, surtout pour l'amélioration de l'adhérence des feuilles adhésives ou des encres d'imprimerie sur ces matériels.
